(19) Europäisches Patentamt / European Patent Office / Office européen des brevets

(11) **EP 4 450 045 A1**

(12) **EUROPEAN PATENT APPLICATION**
published in accordance with Art. 153(4) EPC

(43) Date of publication:
**23.10.2024 Bulletin 2024/43**

(21) Application number: **22906581.8**

(22) Date of filing: **13.12.2022**

(51) International Patent Classification (IPC):
*A61J 3/00* (2006.01)          *A61K 9/08* (2006.01)
*A61K 9/107* (2006.01)        *A61K 9/127* (2006.01)
*A61K 31/4745* (2006.01)    *A61K 41/00* (2020.01)
*A61K 45/00* (2006.01)        *A61P 35/00* (2006.01)
*B82Y 5/00* (2011.01)

(52) Cooperative Patent Classification (CPC):
A61J 3/00; A61K 9/08; A61K 9/107; A61K 9/127;
A61K 31/4745; A61K 41/00; A61K 45/00;
A61P 35/00; B82Y 5/00

(86) International application number:
**PCT/CN2022/138806**

(87) International publication number:
**WO 2023/109836 (22.06.2023 Gazette 2023/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **13.12.2021 CN 202111522472**

(71) Applicant: **Shanghai Best-Link Bioscience, LLC
Shanghai 201203 (CN)**

(72) Inventors:
• **ZHANG, Fuyao
 Shanghai 201203 (CN)**
• **WAN, Jiaxun
 Shanghai 201203 (CN)**

(74) Representative: **Murgitroyd & Company
165-169 Scotland Street
Glasgow G5 8PL (GB)**

(54) **ULTRASONICALLY-ENHANCED CONTINUOUS AND LARGE-SCALE PRODUCTION METHOD FOR NANO-FORMULATIONS**

(57)     Disclosed is an ultrasonically-enhanced continuous and large-scale production method for nano-formulations. Specifically disclosed is a preparation system for continuous production of nano-formulations, comprising (a) a first pipe, (b) a second pipe, (f) an ultrasonic device, (c) a combined pipe and (e) a (fluid) outlet thereof. The first pipe and the second pipe are connected to the combined pipe. A first phase solution enters the combined pipe through a first pipe outlet, and a second phase solution enters the combined pipe through a second pipe outlet. The ultrasonic device acts on the part or the whole of the combined pipe. The first phase solution and the second phase solution are turbulently mixed in the combined pipe to form a combined phase, and flow out through the outlet of the combined pipe. The preparation system and the preparation method of the present invention can remarkably improve the entrap enteficiency of nano-drugs, and improve the uniformity of the particle size of the nano-particles.

Inner diameter of the second pipeline $D_2(IN)$=5.4 mm;
Outer diameter of the second pipeline $D_2(O)$=6 mm;

Ultrasonic bath

Inner diameter of the combined pipeline $D_3(IN)$=5.4 mm;

Combined pipeline

Outer diameter of the combined pipeline $D_3(O)$=6 mm;

First pipeline

Outer diameter of a tail end of the first pipeline $D_1(O)$=2 mm;
Aperture of a spray hole in the tail end of the first pipeline $D_1(S)$=0.3 mm;

Figure 1

**Description**

This application claims priority to Chinese Application No. 202111522472X, filed Dec. 13, 2021. The entire disclosures of the above applications are incorporated herein by reference.

FIELD OF THE INVENTION

The present invention relates to the field of biomedical technology, specifically relates to a continuous, large-scale production system and method for producing nano-formulations. The production method comprises the rapid mixing of two-phase solutions under the action of turbulent shear and ultrasound at the same time, to form a stable nano-formulations with a certain particle size and distribution coefficient. The preparation method has good stability, high reproducibility, and can realize step-by-step scale-up, which is suitable for continuous and large-scale production of nano-formulations.

The present invention belongs to the field of nano drug formulation technology.

BACKGROUND OF THE INVENTION

During the treatment of tumors, there are a variety of therapeutic options to prolong the survival of patients. Anti-tumor drugs used in the treatment include chemotherapy drugs, targeted drugs, photosensitive molecules, photothermal molecules, peptides, proteins, siRNAs, etc. Among them, some drugs need to be made into nano formulations to achieve the anti-tumor effect. Commonly used nano formulations include: nanoparticles, nanoliposomes, polymeric nanomicelles, dendrimers, etc.

The current large-scale production methods of nano formulations include highpressure homogenization method, high-shear emulsification method, microfluidization homogenization method, etc. However, these production methods are batch preparation, which have the disadvantages of large amount of process control parameters, poor inter-batch reproducibility and difficulty in scale-up production.

Compared with the batch production methods, the continuous preparation method of nano formulations can produce uniform nanoparticles with adjustable sizes during continuous operation. Besides, the quality of nano formulations could be monitored in real time during the production process, and the nano formulations meeting the quality standards can be collected in real time. For example, for liposome systems, continuous production can be performed using ethanol injection method. A microfluidic device of Precision NanoSystems in Canada can be used for the continuous production of liposomes (Langmuir, 2012, 28, 3633), the laminar flow of lipid organic phase and aqueous phase were rapidly mixed in a staggered mixer, and liposomes could be produced continuously by parameter optimization. However, since the microfluidic flow channel is usually in the micrometer scale, the fluid flow is in laminar conditions, the single-channel yield is extremely small, and the scale-up production requires multi-channel paralleling. The consistency of each liposome production unit needs to be controlled. The device for continuous preparation of blank liposomes using ethanol injection method reported in the literature (Pharm Res., 2016, 33, 404-416) and patent (CN201680013882) can prepare blank liposomes with low polydispersity index by adjusting the flow velocity of ethanol phase and aqueous phase.

For polymer/drug nanoparticle systems, the continuous production can be achieved by flash nanoprecipitation (FNP) (US10940118B2, CN108137819, CN108542894). Flash nanoprecipitation method is based on the principle of kinetic control and can realize rapid production of nanoparticles by mixing turbulent fluids. This method has the characteristics of high drug loading, short production time (millisecond level), easy control of the size, easy to scale up and continuous production. The principle of preparing polymer/drug nanoparticles by nanoprecipitation method is as follows: the carrier or stabilizer (usually amphiphilic polymers) and hydrophobic drug dissolved in solvent miscible with water to form a homogeneous solution. Then, the solution is rapidly mixed with the anti-solvent (usually water) in a fixed channel. Since the hydrophobic substance is highly supersaturated in the mixed solvent, it is rapidly nucleated in water, and interacts with the polymer (usually an amphiphilic block polymer) at the same time. The polymer encapsulates the nucleating core to form polymer nanoparticles, protect the nanoparticles from reaggregation, thereby forming nanoparticles with good aqueous dispersion (Expert Opin. Drug Deliv., 2009, 6, 865). The production devices used in the flash nanoprecipitation method include: confined impinging jet mixer (CIJM) (Physical Review Letters, 2003, 91, 118301; AIChE Journal, 2003, 49, 2264), multi-inlet vortex mixer (MIVM) (Mol. Pharm., 2013, 10, 4367; Angew. Chem. Int. Ed. Engl., 2021, 60, 15590).

However, when the existing flash nanoprecipitation (FNP) method is used to continuous preparation of nanoparticles of hydrophobic drugs, the encapsulation efficiency of the hydrophobic drugs decreases significantly and the particle size distribution coefficient of the nanoparticles increases during the scale-up preparation of the nano-formulations. These issues result in poor reproducibility and difficulties in the quality control of the nano-formulations, and increases the risk in the scale-up production of nano-formulations. In addition, although there are existing technologies that use ultrasound combined with microfluidic technology in a non-turbulent state to prepare blank liposomes to reduce their particle size, blank liposomes do not have drug encapsulation efficiency parameter. Therefore, how to improve the encapsulation efficiency of hydrophobic drugs in the process of scale-up preparation of nano-formulations is still an urgent problem needs to be solved.

DETAILED DESCRIPTION

The present invention is intended to overcome the defects and shortcomings of the prior art mentioned above. After repeated research and experiments, we found that the mixing of two-phase solution under turbulent shear and ultrasonic condition at the same time could solve the problem of the decline of drug encapsulation efficiency and the poor particle size uniformity that occurs in the large-scale production of nano-formulations. We provide equipment for continuous, large-scale and controllable production of nano-formulations, method for continuous and large-scale production method of nano-formulations enhanced by ultrasound, and realized the continuous and controllable large-scale production of nano-formulations.

The first purpose of the present invention is to provide equipment for continuous, large-scale and controllable production of nanoparticles, which can promote the formation of nanoparticles, improve the encapsulation efficiency of nanomedicines, prevent the deposition of hydrophobic drug particles on the tube wall, and improve the uniformity of the particle size of nanoparticles. The use of this equipment includes, but is not limited to, the production of nano-formulations with a particle size range of 1~1000 nm. The nano-formulations are polymeric nanomicelles, nanoliposomes, and small molecule nanoparticle assemblies.

The second purpose of the present invention is to provide a method for continuous, large-scale and controllable production of nano-formulations.

The present invention provides a continuous, large-scale and controllable production system for nano-formulations, which includes (a) a first pipeline, (b) a second pipeline, (f) an ultrasonic device, (c) a combined pipeline and (e) a (fluid) outlet;

Wherein, the first pipeline and the second pipeline are connected to the combined pipeline, the first pipeline is coaxial with the combined pipeline and the second pipeline is perpendicular to the combined pipeline. The first pipeline outlet is positioned within the combined pipeline. The first phase solution enters the combined pipeline through the first pipeline outlet, the second phase solution enters the combined pipeline through the second pipeline outlet. The ultrasonic device acts on part or the whole of combined pipeline. The first phase solution and the second phase solution are mixed in the combined pipeline to form a combined phase; the combined phase flows out through the outlet of the combined pipeline.

In some embodiments, the nano-formulations are selected from one of polymeric nanomicelles, polymer nanoparticles, nanoliposomes, lipid nanoparticles and small molecule nanoparticle assemblies.

In some embodiments, the features of the production system include:

(1) The core part of the production system includes: (a) a first pipeline; (b) a second pipeline; (c) a combined pipeline; (d) turbulent mixing device; (e) fluid outlet; (f) ultrasound devices with adjustable power;
Wherein, the first pipeline and the second pipeline are connected to the combined pipeline, the first phase solution enters the combined pipeline through the first pipeline outlet, the second phase solution enters the combined pipeline through the second pipeline outlet, the first phase solution and the second phase solution are mixed in the combined pipeline to form a combined phase. The power adjustable ultrasonic device acts on part or the whole of combined pipeline. The combined phase is fully mixed by a turbulent mixing device. After the mixing, the nanoparticles are collected into a suitable container through the outlet of the combined pipeline.
(2) The mixing process of the first phase solution and the second phase solution is carried out under ultrasonication;
(3) The nano-formulations are selected from one of polymeric nanomicelles, nanoliposomes or small molecule nanoparticle assemblies.

In some embodiments, the mixing is turbulent mixing. The turbulent mixing can be achieved by adding a turbulent mixing device in the combined pipeline. There can be one or more turbulent mixing devices.

In some embodiments, the first pipeline outlet is a spray hole with a certain shape and diameter, and the first phase solution passes through the first pipeline and enters the combined pipeline through the spray hole.

In some embodiments, the spray hole diameter D1(S) at the end of first pipeline is selected from 0.03-5.0 mm; the second pipeline inner diameter D2(IN) is selected from 0.3-50.0 mm; the combined pipeline inner diameter D3(IN) is selected from 0.3-50.0 mm.

In some embodiments, the combined pipeline length (i.e., combined phase length) is selected from 6 to 120 cm, such as 9 cm or 36 cm.

In some embodiments, the ratio of the combined pipeline length to the combined pipeline inner diameter is (16-450):1, such as 16.7:1, 30:1 or 450:1.

In some embodiments, the first pipeline outer diameter D1(O) is 0.35 to 2 mm, such as 0.35 mm, 1 mm or 2 mm.

In some embodiments, the spray hole diameter D1(S) at the end of first pipeline is 0.2 to 0.6 mm, such as 0.2 mm, 0.25 mm, 0.3 mm, 0.4 mm or 0.6 mm.

In some embodiments, the second pipeline outer diameter D2(O) is 6 mm.

In some embodiments, the second pipeline inner diameter D2(IN) is 0.8 to 5.4 mm, such as 0.8 mm, 3.0 mm or 5.4 mm.

In some embodiments, the combined pipeline outer diameter D3(O) is 6 mm.

In some embodiments, the combined pipeline inner diameter D3(IN) is 0.8 to 5.4 mm, such as 0.8 mm, 3.0 mm or 5.4 mm.

In some embodiments, the second pipeline inner diameter D2(IN) is the same as the combined pipeline inner diameter D3(IN).

In some embodiments, the ratio of the spray hole diameter D1(S) at the end of first pipeline to the combined pipeline inner diameter D3(IN) can be 1:(2-50), such as 1:3.2, 1:7.5, 1:9 or 1:18.

In some embodiments, the turbulent mixing device is a device that mixes the first phase solution and the second phase solution to reach a turbulent flow state, such as a static mixer. The static mixer can be selected from one or more of SV type static mixer, SX type static mixer, SL type static mixer, SH type static mixer and SK type static mixer, preferably SK type static mixer.

In some embodiments, the materials used in the first pipeline, the second pipeline, the combined pipeline, the turbulent mixing device, and the fluid outlet are each selected from one or more of stainless steel, polytetrafluoroethylene, polyethylene, polypropylene, latex, silicone, or other polymer materials.

In some embodiments, the turbulent mixing can cause the fluid in the combined phase to reach a turbulent transition state or turbulent flow state by increasing the fluid flow velocity. The Reynolds number in the combined phase depends on the smoothness of the circular stainless steel pipe wall. For example, when the pipe wall is rough, a lower Reynolds number can also achieve turbulent mixing conditions (such as Re between 500-2000, this range is usually considered to be a laminar flow conditions).

In some embodiments, the turbulent mixing can be achieved by making the combined phase a bent pipe with a certain curvature, changing the direction of fluid flow, enhancing the convection of the fluid, and enhancing the mixing of the fluid. At this time, in addition to being greater than 4000, the Reynolds number in the combined phase calculated based on the fluid in the circular tube can also be between 500-4000.

In some embodiments, the turbulent mixing can be achieved by equipping a static mixer in the combined phase. The static mixer can include but is not limited to: SV type static mixer, SX type static mixer, SL type static mixer, SH type static mixers, SK type static mixers, etc. that can divide the fluid through turbulent mixing elements, change the flow direction of the fluid, enhance the convection of the fluid, and increase the mixing of the fluid. At this time, in addition to being greater than 4000, the Reynolds number in the combined phase calculated based on the fluid in the circular tube can also be between 500-4000.

In some embodiments, the quantity of flow Q1 of the first phase solution through the first pipeline is selected from 1-1000 ml/min; the temperature t1 of the first phase solution is selected from 0-90°C; the quantity of flow Q2 of the second phase solution through the second pipeline is selected from 10-10000 ml/min; the temperature t2 of the second phase solution is selected from 0-90°C.

In some embodiments, the ultrasonic frequency of the power adjustable ultrasonic device is 15 kHz~1.0 MHz, the ultrasonic power range is 0.1-20 kW.

In some embodiments, the ultrasonic frequency of the power adjustable ultrasonic device is 15 kHz~40 kHz, and the ultrasonic power range is 0.1-20 kW.

In some embodiments, the ultrasonic frequency of the power adjustable ultrasonic device is 15 kHz~40 kHz, and the ultrasonic power range is 100-1000 W.

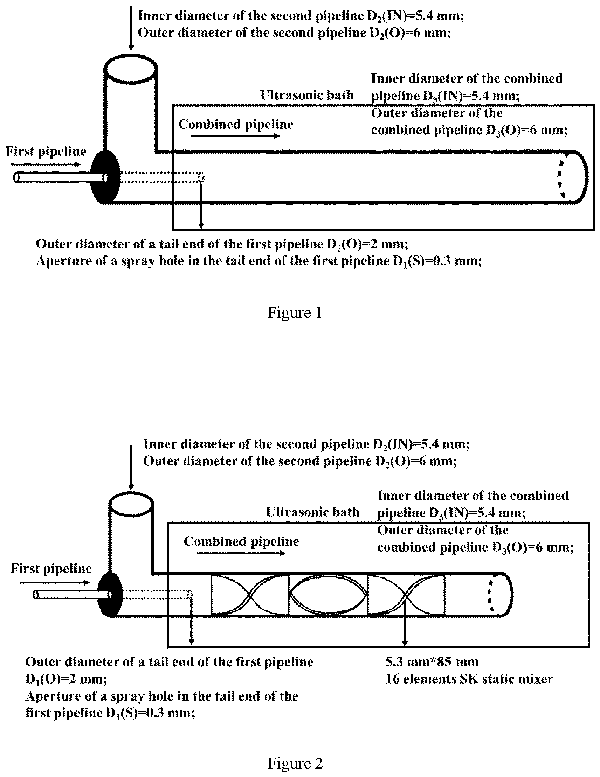

In some embodiments, the structure of the production system is shown in Figure 1 or Figure 2.

In some embodiments, the polymeric nanomicelle is polymeric nanomicelle encapsulating antitumor drug, wherein the polymeric nanomicelle component is selected from an amphiphilic polymer and an antitumor drug; the amphiphilic polymer is selected from PEG-PLA, PEG-PCL, PEG-linker-PLA, PEG-linker-PCL, wherein the linker is selected from C1-C30 small molecule fragment; and the average molecular weight of PEG is 400-20000 polyethylene glycol segments or mono-protected polyethylene glycol segments.

In some embodiments, the nanoliposomes are blank liposomes without drug encapsulation.

In some embodiments, the nanoliposomes are liposomes encapsulating antitumor drugs.

In some embodiments, the lipid nanoparticles are lipid nanoparticles encapsulating antitumor drugs.

In some embodiments, the small molecule nanoassemblies are selected from antitumor drug/photosensitizer nanoassemblies, antitumor drug/antitumor drug nanoassemblies, antitumor drug/other drug (e.g., curcumin) nanoassemblies, antitumor drug/excipient (e.g., amphiphilic polymer PEG-PLA, DSPE-PEG or PLGA polymer) nanoassemblies, two or more drug nanoassemblies (e.g., SN38 and irinotecan), or small molecule drug/excipient nanoassemblies.

In some embodiments, the antitumor drug is selected from one or more of abemaciclib, abiraterone, abrocitinib, acalabrutinib, afatinib, aldesleukin, alectinib, alflutinib, almonertinib, altretamine, amcenestrant, aminoglutethimide, amsacrine, anastrozole, anlotinib, apalutamide, apatinib, arzoxifene, asciminib, asparaginase, avapritinib, avitinib, axitinib, azacitidine, baricitinib, belinostat, bendamustine, bexarotene, bicalutamide, bicyclol, binimetinib, bleomycin, boanmycin, bortezomib, bosutinib, brigatinib, buserelin, busulfan, cabazitaxel, cabozantinib, calaspargase, calicheamycin, capecitabine, capmatinib, carboplatin, carfilzomib, carmustine, carmofur, cedazuidine, ceritinib, cetrorelix, chidamide, chlorambucil, cisplatin, cladribine, clofarabine, cobimetinib, colchicine, copanlisib, crizotinib, cyclophosphamide, cytarabine, dabrafenib, dacarbazine, dacomitinib, dactinomycin, dalpiciclib, darolutamide, dasatinib, daunorubicin, decitabine, degarelix, delgociclib, denileukin, deruxtecan, docetaxel, donafenib, doxorubicin, duvelisib, enasidenib, encorafenib, ensartinib, entrectinib, enzalutamide, enzastaurin, elacestrant, epirubicin, erdafitinib, eribulin, erlotinib, estradiol, estramustine, etoposide, everolimus, exemestane, fasudil, fedatinib, filgotinib, floxuridine, fludarabine, flumatinib, fluorouracil, flutamide, fluzoparib, formestane, fostamatinib, fruquintinib, fulvestrant, gefitinib, gemcitabine, gilteritinib, giredestrant, glasdegib, goserelin, histrelin, hydroxyurea, ibrutinib, ibudilast, icaritin, icotinib, idarubicin, idelalisib, ifosfamide, imatinib, imiquimod, infigratinib, ingenol mebutate, interferon alfa-2b, irinotecan, ivosidenib, ixabepilone, ixazomib, lanreotide, lapatinib, larotrectinib, lenalidomide, lenvatinib, letrozole, leucovorin, leuprolide, lomustine, lonafarnib, lorlatinib, lurbinctedin, maytansine, mechlorethamine, medroxyprogesterone, megestrol, melphalan, melphlan flufenamide, mercaptopurine, methotrexate, methoxsalen, methylprednisolone, midostaurin, mitomycin, mitotane, mitoxantrone, mitozolomide, mobocertinib, monomethylauristatin E, monomethylauristatin F, nelarabine, nandrolone, neratinib, nearsudil, nilotinib, nilutamide, nintedanib, niraparib, octreotide, olaparib, olmutinib, omacetaxine, orelabrutinib, osimertinib, oxaliplatin, paclitaxel, pacritinib, palbociclib, pamidronate, pamiparib, panobinostat, pazopanib, peficitinib, pegaptanib, pegaspargase, peginteferon alfa-2b, pemigatinib, pemetrexed, pentetreotide, pentostatin, pexidartinib, phenoxybenzamine, pidotimod, plinabulin, plitidepsin, pomalidomide, ponatinib, porfimer, pralatrexate, pralsetinib, prednisolone, procarbazine, pyrotinib, quizartinib, radotinib, raloxifene, raltitrexed, regorafenib, ribociclib, rintatolimod, ripretinib, romidepsin, rucaparib, ruxolitinib, savolitinib, selinexor, selpercatinib, selumetinib, sonidegib, sorafenib, sotorasib, streptozocin, sunitinib, surufatinib, talazoparib, tamoxifen, tazemetostat, tegafur, temozolomide, temsirolimus, teniposide, tepotinib, teprenone, thalidomide, thioguanine, thiotepa, thyrotropin alfa, tipiracil, tipifamib, tirabrutinib, tirbanibulin, tivozanib, trametinib, tofacitinib, topotecan, toremifene, trabectedin, tretinoin, trifluride, trilaciclib, triptorelin, tucatinib, upadacitinib, umbralisib, utidelone, uroacitide, valrubicin, vandetanib, vemurafenib, venetoclax, vinblastine, vincristine, vindesine, vinflunine, vinorelbine, vismodegib, vorinostat, zanubrutinib, zoledronic acid, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, 7-N,N-dimethylglycylpaclitaxel, 7-L-alanylacetaxel, larotaxel, camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, pirarubicin, aclacinomycin, sirolimus, tacrolimus, progesterone, estrogen, rapamycin, mithramycin, harringtonine or curcumin;

Furthermore, the antitumor drug is preferably selected from one or more of camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, topotecan, deruxtecan, paclitaxel, docetaxel, cabazitaxel, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, 7-N,N-dimethylglycylpaclitaxel, 7-L-alanylacetaxel, larotaxel, doxorubicin, epirubicin, daunorubicin, pirarubicin, aclacinomycin, etoposide, teniposide, vinblastine, vincristine, vinorelbine, vindesine, maytansine, curcumin, harringtonine, homoharringtonine, gemcitabine, capecitabine, fludarabine, cladribine, pemetrexed, bortezomib, carfilzomib, ixazomib, carmustine, fluorouracil, cytarabine, cyclosporine A, eribulin, trabectedin, gefitinib, erlotinib, lapatinib, afatinib, dacomitinib, vandetanib, neratinib, osimertinib, imatinib, sorafenib, sunitinib, lapatinib, dasatinib, olaparib, niraparib, rucaparib, fluzoparib, pamiparib, veliparib, talazoparib, apatinib, palbociclib, abemaciclib, ribociclib.

In some embodiments, the photosensitizers include cyanine molecules, porphyrin molecules, porphyrin precursors, phthalocyanine molecules and chlorin molecules; wherein, the cyanine molecules are preferably selected from one or more of indocyanine green (IR780), new indocyanine green (IR820), indocyanine green and indocyanine green analogs; the porphyrin molecule is preferably selected from hematoporphyrin monomethyl ether; the porphyrin precursor is preferably selected from one of 5-aminolevulinic acid and/or 5-aminolevulinic acid esters; the phthalocyanine molecule is preferably selected from one or more of copper phthalocyanine, cobalt phthalocyanine, aluminum phthalocyanine, nickel phthalocyanine, calcium phthalocyanine, sodium phthalocyanine, magnesium phthalocyanine, zinc phthalocyanine, indium phthalocyanine, oxytitanium phthalocyanine, manganese phthalocyanine or phthalocyanine derivatives; the chlorin molecules are preferably selected from one or more of chlorins, talaporfin, verteporfin, temoporfin, rostaporfin, porfimer sodium, hemoporfin and HPPH.

In some embodiments, the polymeric nanomicelles are PTX/PEG-PLA polymeric micelles or PTX/PEG-Phe-PLA polymeric micelles.

In some embodiments, the nanoliposomes are HSPC/CHOL/DSPE-PEG blank liposomes or PTX/HSPC/CHOL/DSPE-PEG liposomes.

In some embodiments, in the nanoliposomes, the molar ratio of HSPC/CHOL/DSPE-PEG is 56:38:5 or 89:57:4.

In some embodiments, the lipid nanoparticle is a PolyI lipid nanoparticle, such as PolyI/ALC-0315/DSPE-PEG2000/HSPC/cholesterol.

In some embodiments, the polymer nanoparticles are PEG-PLA polymer nanoparticles, PLGA polymer nanoparticles or PTX/PLGA polymer nanoparticles.

In some embodiments, the small molecule nanoassembly is an anti-tumor drug/photosensitizer nanoassembly, preferably SN-38/ICG nanoparticles, PTX/ICG nanoparticles, curcumin/CPT11 nanoparticles or SN-38/CPT11 nanoparticles.

In some embodiments, the drug loading of anti-tumor drugs is 10%-90%, such as 10%, 15%, 20%, 25%, 30%, 35%, 40%, 45%, 50%, 55%, 60%, 65%, 70%, 75%, 80%, 85%, 90%.

The present invention also provides a production method for producing nano-formulations, which includes the following steps: in the production system as described above, the first phase solution and the second phase solution are mixed under ultrasonication, nano-formulations are collected from combined phase through the fluid outlet;

A. When the nano-formulations are polymeric nanomicelle, the solvent in the first phase solution is a good solvent for an anti-tumor drug or its pharmaceutically acceptable salt, and the solute is (1) the anti-tumor drug or its pharmaceutically acceptable salt, or (2) the anti-tumor drug or its pharmacologically acceptable salt and polymer;

The solvent in the second phase solution is an anti-solvent of the anti-tumor drug or its pharmaceutically acceptable salt, and the solute is (1) absent, or (2) polymer;
When the solute in the first phase solution is an anti-tumor drug or its pharmaceutically acceptable salt and polymer, the solute in the second phase solution does not exist;
When the solute in the first phase solution is an anti-tumor drug or its pharmaceutically acceptable salt, the solute in the second phase solution is polymer;

B. When the nano-formulations are polymer nanoparticles, the solvent of the first phase solution is a good solvent for the polymer, and the solute is (1) polymer, or (2) polymer and anti-tumor drug;
The second phase solution is water or water containing 0.5% PVA;
C. When the nano-formulations are nanoliposomes, the solvent of the first phase solution is a good solvent for the lipid component, and the solute is (1) the lipid component of the liposome, or (2) the lipid component of the liposome and the anti-tumor drug;
The second phase solution is water or aqueous buffer solution with a certain pH value and a certain osmotic pressure;
D. When the nano-formulations are lipid nanoparticle, the solvent of the first phase solution is a good solvent for the lipid component, and the solute is the lipid component of the lipid nanoparticle;
The solvent of the second phase solution is aqueous buffer solution with a certain pH value and a certain osmotic pressure; The solute is anti-tumor drug;
E. When the nano-formulations are anti-tumor drug/photosensitizer nanoassembly, the solvent in the first-phase solution is a good solvent of the anti-tumor drug or its pharmaceutically acceptable salt, and the solute is (1) the anti-tumor drug or its pharmaceutically acceptable salt and the photosensitizer, or (2) the anti-tumor drug or its pharmaceutically acceptable salt;

The solvent in the second phase solution is an anti-solvent of the anti-tumor drug or its pharmaceutically acceptable salt, and the solute is (1) absent, or (2) a photosensitizer;
When the solute in the first phase solution is an anti-tumor drug or its pharmaceutically acceptable salt and a photosensitizer, the solute in the second phase solution does not exist;
When the solute in the first phase solution is an anti-tumor drug or its pharmaceutically acceptable salt, the solute in the second phase solution is a photosensitizer.

In some embodiments, the temperature of the first phase solution is 0-90°C, such as 25°C or 60°C.

In some embodiments, the temperature of the second phase solution is 0-90°C, such as 25°C or 60°C.

In some embodiments, the fluid Reynolds number Re of the combined phase is 700-9500 (e.g., 747, 2884, 3868, 5158, 5505, 5872, 6623, 7865 or 9176), preferably 3000-7000 (e.g., 3868, 5158 or 6623).

In some embodiments, the flow velocity ratio FVR between the first phase solution and the combined phase is 0.4-6, such as 0.49, 0.64, 0.93, 1.46, 3.38, 3.4, 4.4 or 5.2.

In some embodiments, the quantity of flow $Q_1$ of the first phase solution through the first pipeline is selected from 10-100 ml/min, such as 10, 11, 14, 50, 60, 80 or 100.

In some embodiments, the quantity of flow Q2 of the second phase solution through the second pipeline is selected from 100-1300 ml/min, such as 100, 193, 200, 210, 300, 936, 890 or 1248.

In some embodiments, the ultrasound is an ultrasonic water bath, and the ultrasonic power is 200W.

In some embodiments, the present invention also provides a method for preparing polymeric micelles, and the method includes: one or more of the anti-tumor drugs or their pharmaceutically acceptable salts are dissolved in a first phase solvent, and the solvent for the first phase solution is a good solvent for the anti-tumor drugs or their pharmaceutically acceptable salts. One or more of polymers are dissolved in a second phase solvent, and the solvent for the second phase solution is an anti-solvent for anti-tumor drugs or their pharmaceutically acceptable salts. The first phase solution with the quantity of flow Q1 and the second phase solution with the quantity of flow Q2 are mixed in the combined phase. Under the action of turbulent shear and ultrasound at the same time, the two-phase solutions mix rapidly to form a mixed solvent of the first phase and the second phase and produce a stably dispersed polymeric micelles encapsulating anti-tumor drug with a certain particle size and distribution coefficient. In the process of mixing the two-phase solution, the deposition of hydrophobic drug particles on the tube wall can be prevented by ultrasound, which ensures the stability and controllability of the scale-up production of nano-formulations.

In some embodiments, the present invention also provides a method for preparing polymeric micelles, and the method includes: one or more of the anti-tumor drugs or their pharmaceutically acceptable salts and one or more of polymers are dissolved in a first phase solvent, and the solvent for the first phase solution is a good solvent for the anti-tumor drugs or their pharmaceutically acceptable salts. The solvent for the second phase solution is an anti-solvent for anti-tumor drugs or their pharmaceutically acceptable salts. The first phase solution with the quantity of flow Q1 and the second phase solution with the quantity of flow Q2 are mixed in the combined phase. Under the action of turbulent shear and ultrasound at the same time, the two-phase solutions mix rapidly to form a mixed solvent of the first phase and the second phase and produce a stably dispersed polymeric micelles with a certain particle size and distribution coefficient. In the process of mixing the two-phase solution, the deposition of hydrophobic drug particles on the tube wall can be prevented by ultrasound, which ensures the stability and controllability of the scale-up production of nano-formulations.

In some embodiments, the concentration of the anti-tumor drug in the first phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml, more preferably 10-20 mg/ml, such as 15 mg/ml.

In some embodiments, the concentration of the polymer in the first phase solution or the second phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml, such as 50 mg/ml.

In some embodiments, the present invention also provides a method for preparing nanoliposomes, and the method includes: the lipid component of the liposome is dissolved in the first phase, and the solvent used in the first phase solution is a good solvent for the lipid component. The second phase solution is water, aqueous buffer solution with a certain pH value and a certain osmotic pressure. The first phase solution with the quantity of flow Q1 and the second phase solution with the quantity of flow Q2 are mixed in the combined phase. Under the action of turbulent shear and ultrasound at the same time, the two-phase solutions mix rapidly to form a mixed solvent of the first phase and the second phase and produce a stably dispersed blank liposomes with a certain particle size and distribution coefficient. In the process of mixing the two-phase solution, the deposition of particles on the tube wall can be prevented by ultrasound, which ensures the stability and controllability of the scale-up production of nano-formulations.

In some embodiments, the concentration of the lipid component in the first phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml.

In some embodiments, when the lipid component is HSPC/CHOL/DSPE-PEG2k, the concentration of the HSPC in the first phase solution is 10 mg/ml.

In some embodiments, the present invention also provides a method for preparing anti-tumor drug/photosensitizer nanoassembly, and the method includes: one or more of the anti-tumor drugs or their pharmaceutically acceptable salts are dissolved in a first phase solvent. One or more of photosensitizers are dissolved in a second phase solvent, and the solvent for the second phase solution is an anti-solvent for anti-tumor drugs or their pharmaceutically acceptable salts. The first phase solution with the quantity of flow Q1 and the second phase solution with the quantity of flow Q2 are mixed in the combined phase. Under the action of turbulent shear and ultrasound at the same time, the two-phase solutions mix rapidly to form a mixed solvent of the first phase and the second phase and produce a stably dispersed anti-tumor drug/photosensitizer nanoassembly with a certain particle size and distribution coefficient. In the process of mixing the two-phase solution, the deposition of hydrophobic drug particles on the tube wall can be prevented by ultrasound, which ensures the stability and controllability of the scale-up production of nano-formulations.

In some embodiments, the present invention also provides a method for preparing anti-tumor drug/photosensitizer nanoassembly, and the method includes: one or more of the anti-tumor drugs or their pharmaceutically acceptable salts and one or more of the photosensitizers are dissolved in a first phase solvent, and the solvent for the first phase solution is a good solvent for the anti-tumor drugs or their pharmaceutically acceptable salts. The solvent for the second phase solution is an anti-solvent for anti-tumor drugs or their pharmaceutically acceptable salts. The first phase solution with the quantity of flow Q1 and the second phase solution with the quantity of flow Q2 are mixed in the combined phase. Under the action of turbulent shear and ultrasound at the same time, the two-phase solutions mix rapidly to form a mixed solvent of the first phase and the second phase and produce a stably dispersed anti-tumor drug/photosensitizer nanoassembly with a certain particle size and distribution coefficient. In the process of mixing the two-phase solution, the deposition of hydrophobic drug particles on the tube wall can be prevented by ultrasound, which ensures the stability and controllability of the scale-up production of nano-formulations.

In some embodiments, the molar ratio of the anti-tumor drug or its pharmaceutically acceptable salt to the photosensitizer is (1-15):1, such as 1:1, 2:1, 5:1, 6:1, 7:1, 8:1, 10:1 or 15:1, preferably 2:1.

In some embodiments, the concentration of the anti-tumor drug in the first phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml, such as 40 mg/ml, 50 mg/ml or 100 mg/ml.

In some embodiments, the concentration of the photosensitizer in the first phase solution or the second phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml, such as 40 mg/ml, 50 mg/ml or 150 mg/ml.

In some embodiments, the solvent used in the first phase solution and the second phase solution is water, aqueous buffer solution with a certain pH value, or an organic solvent miscible with water. Further, the organic solvent is one or more of methanol, ethanol, propanol, isopropanol, butanol, isobutanol, tert-butanol, DMF, DMAc, HMPA, N-methylpyrrolidone, DMSO, butyl sulfone, tetramethylene sulfone, THF, 2-methyltetrahydrofuran, acetonitrile, acetone, ethylene glycol, ethylene glycol methyl ether, ethylene glycol ethyl ether, dioxane, formic acid, acetic acid, hydroxypropionic acid, ethylamine, ethylenediamine, glycerol or pyridine.

In some embodiments, when the nano-formulation is polymeric nanomicelle, the solvent used in the first phase solution is nitrile solvent or alcohol solvent, such as acetonitrile or ethanol.

In some embodiments, when the nano-formulation is polymeric nanomicelle, the solvent used in the second phase solution is water.

In some embodiments, when the nano-formulation is liposome, the solvent used in the first phase solution is alcohol solvent, such as ethanol.

In some embodiments, when the nano-formulation is liposome, the solvent used in the second phase solution is water or aqueous solution of ammonium sulfate, preferably water or an aqueous solution of 120 mM ammonium sulfate.

In some embodiments, when the nano-formulation is polymer nanoparticle, the solvent used in the first phase solution is alcohol solvent or chlorinated alkane solvent, such as ethanol or methylene chloride.

In some embodiments, when the nano-formulation is polymer nanoparticle, the solvent used for the second phase solution is water or water containing 0.5% PVA.

In some embodiments, when the nano-formulation is anti-tumor drug/photosensitizer nanoassembly, the solvent used in the first phase solution is sulfoxide solvent, alcohol solvent, such as dimethyl sulfoxide or methanol.

In some embodiments, when the nano-formulation is anti-tumor drug/photosensitizer nanoassembly, the solvent used in the second phase solution is water.

In some embodiments, when the nano-formulation is lipid nanoparticle, the first phase solution is alcohol solvent, such as ethanol.

In some embodiments, when the nano-formulation is lipid nanoparticle, the second phase solution is a citrate buffer solution (pH 4.0).

In some embodiments, the particle size of nano-formulations is less than 1000 nm.

In some embodiments, the particle size of nano-formulations is less than 500 nm.

In some embodiments, the particle size of nano-formulations is less than 200 nm.

In some embodiments, the particle size of nano-formulations is selected from 20-200 nm.

In some embodiments, the polydispersity index of nano-formulations is less than 0.3.

In some embodiments, the polydispersity index of nano-formulations is less than 0.2.

In some embodiments, the polydispersity index of nano-formulations is less than 0.1.

Further, the present invention also provides a preparation method for the continuous production of SN-38/indocyanine green nanoassemblies, and the method includes: SN-38 and indocyanine green are dissolved in the first phase solution, wherein the solvent used in the first phase solution is a good solvent for SN-38 and indocyanine green, and the second phase solution is an anti-solvent for anti-tumor drugs or their pharmaceutically acceptable salts. The first phase solution with the quantity of flow Q1 and the second phase solution with the quantity of flow Q2 are mixed in the combined phase. Under the action of turbulent shear and ultrasound at the same time, the two-phase solutions mix rapidly to form a mixed solvent of the first phase and the second phase and produce a stably dispersed SN-38/indocyanine green nanoassembly with a certain particle size and distribution coefficient. The solvent used in the first phase solution and the second phase solution is water, aqueous buffer solution with a certain pH value or an organic solvent miscible with water. Further, the organic solvent is one or more of methanol, ethanol, propanol, isopropanol, butanol, isobutanol, tert-butanol, DMF, DMAc, HMPA, N-methylpyrrolidone, DMSO, butyl sulfone, tetramethylene sulfone, THF, 2-methyltetrahydrofuran, acetonitrile, acetone, ethylene glycol, ethylene glycol methyl ether, ethylene glycol ethyl ether, dioxane, formic acid, acetic acid, hydroxypropionic acid, ethylamine, ethylenediamine, glycerol or pyridine.

In some embodiments, SN-38/ICG nanoparticles prepared at a scale of 15-20 ml without ultrasonication had an encapsulation efficiency of 98.4% and a nanoparticle size of $98\pm4$ nm.

In some embodiments, the encapsulation efficiency of SN-38/ICG nanoparticles prepared at a scale of 1.0 L without ultrasonication decreased to 92.4%. There was obvious SN-38 drug deposition in the pipeline outlet, and the particle size of nanoparticles was $111\pm6$ nm.

In some embodiments, the encapsulation efficiency of SN-38/ICG nanoparticles prepared at a scale of 1.0 L under ultrasonic conditions was close to that of small batch preparation, which was 97.5%, and the average diameter was $109\pm10$ nm.

In some embodiments, SN-38/ICG nanoparticles prepared at a scale of 15-20 ml without ultrasonication had an encapsulation efficiency of 98.8% and a nanoparticle size of $109\pm5$ nm.

In some embodiments, the encapsulation efficiency of SN-38/ICG nanoparticles prepared at a scale of 425 mL without ultrasonication decreased to 92.2%. There was obvious SN-38 drug deposition in the pipeline outlet, and the particle size of nanoparticles was $126\pm4$ nm.

In some embodiments, the encapsulation efficiency of SN-38/ICG nanoparticles prepared at a scale of 425 mL under ultrasonic conditions was close to that of small batch preparation, which was 97.0%, and the average diameter was $113\pm4$ nm.

In some embodiments, the PTX/ICG nanoparticles prepared at a scale of 20 ml without ultrasonication had a PTX encapsulation efficiency of 98.2% and a nanoparticle size of $75\pm3$ nm.

In some embodiments, the PTX encapsulation efficiency of PTX/ICG nanoparticles prepared at a scale of 400 ml without ultrasonication was reduced to 93.4%, and the particle size of the nanoparticles was $81\pm5$ nm.

In some embodiments, the PTX encapsulation efficiency of PTX/ICG nanoparticles prepared at a scale of 400 ml under ultrasonic conditions is close to that of small batch preparation, which is 98.7%, and the average diameter is $72\pm2$ nm.

In some embodiments, HSPC/CHOL/DSPE-PEG blank liposomes were prepared without ultrasonication, and the blank liposomes obtained showed a distribution with multiple peaks.

In some embodiments, the HSPC/CHOL/DSPE-PEG blank liposomes prepared under ultrasonic conditions showed a distribution with single peak. The particle size was $131.7\pm2.9$ nm, indicating that ultrasound was able to promote the formation of blank liposomes.

In some embodiments, the HSPC/CHOL/DSPE-PEG blank liposomes prepared under ultrasonic conditions showed a distribution with single peak. The particle size was $115.3\pm1.6$ nm, indicating that ultrasound was able to promote the formation of blank liposomes.

In some embodiments, HSPC/CHOL/DSPE-PEG/Paclitaxel liposomes prepared under ultrasonic conditions showed a distribution with single peak. The particle size was $81.1\pm1.7$ nm, indicating that ultrasound could promote the formation of paclitaxel liposomes.

In some embodiments, the PTX/PEG-PLA polymeric micelles prepared without ultrasonic conditions have many white solids in the solution, wide particle size distribution and poor reproducibility.

In some embodiments, the PTX/PEG-PLA polymeric micelles prepared under ultrasonication conditions were uniformly distributed in particle size, with an average diameter of $25.6\pm1.0$ nm, indicating that ultrasound could promote the formation of drug-loaded polymeric micelles.

In some embodiments, the PTX/PEG-Phe-PLA polymeric micelles prepared without ultrasonic conditions have many white solid in the solution, wide particle size distribution and poor reproducibility.

In some embodiments, PTX/PEG-Phe-PLA polymeric micelles prepared under ultrasonic conditions had uniform particle size distribution, with an average diameter of $23.4\pm0.8$ nm, indicating that ultrasound could promote the formation of drug-loaded polymeric micelles.

Compared with the prior art, the present invention has the following benefits: the producing system and producing method of the present invention can significantly improve the encapsulation efficiency of nanomedicine, and improve the uniformity of particle size of nanoparticles. In addition, the deposition of the drug is reduced due to the increased encapsulation efficiency. It is conducive to continuous preparation and production.

DESCRIPTION OF FIGURES

Figure 1: Schematic diagram of nano-formulation preparation device without static mixer

Figure 2: Schematic diagram of nano-formulation preparation device with SK static mixer in the combined phase

Figure 3: Example 4 particle size distribution of SN-38/ICG nanoparticles prepared under low turbulence conditions with/without ultrasonication

Figure 4: Example 5 SN-38/ICG nanoparticle size distribution diagram, from which it can be concluded that when SN-38/ICG is prepared in small quantities (10-25mL), the nanoparticles obtained by preparation device without ultrasonication show a distribution with single peak, and the average particle size is $98\pm4$ nm.

Figure 5: Example 6 SN-38/ICG nanoparticle size distribution diagram, from which it can be concluded that when SN-38/ICG is prepared in large quantities (1.0 L), the nanoparticles obtained by preparation device without ultrasonication show a distribution with double peaks, and the average particle size is $111\pm6$ nm.

Figure 6: Example 7 SN-38/ICG nanoparticle size distribution diagram, from which it can be concluded that when SN-38/ICG is prepared in large quantities (1.0 L), the nanoparticles obtained under ultrasonication show a distribution with single peak, and the average particle size is $109\pm10$ nm.

Figure 7: Example 20 and Example 21 particle size distribution diagram of HSPC/CHOL/DSPE-PEG blank liposomes, from which it can be concluded that when the HSPC/CHOL/DSPE-PEG blank liposomes are prepared without ultrasonication, the obtained blank liposomes show a distribution with multiple peaks.

Figure 8: Example 22 and Example 23 HSPC/CHOL/DSPE-PEG blank liposome particle size distribution diagram, from which it can be concluded that when the HSPC/CHOL/DSPE-PEG blank liposomes are prepared under ultrasonication, the obtained blank liposomes show a distribution with single peak. The particle sizes are $131.7\pm2.9$ nm and $115.3\pm1.6$ nm, respectively.

Figure 9: Example 24 PTX/HSPC/CHOL/DSPE-PEG liposome particle size distribution diagram, from which it can be concluded that when PTX/HSPC/CHOL/DSPE-PEG liposomes are prepared under ultrasonication, the obtained paclitaxel liposomes show a distribution with single peak, and the particle size is $81.1\pm1.7$nm.

Figure 10: Example 27 PTX/PEG-PLA nanomicelle particle size distribution diagram, from which it can be concluded that when PTX/PEG-PLA nanomicelles are prepared under ultrasonication, the obtained nanomicelles show a distribution with single peak, and the particle size is $45.1\pm1.1$ nm.

Figure 11: Example 30 PEG-PLA nanoparticle size distribution diagram, from which it can be concluded that when PEG-PLA nanoparticles are prepared without ultrasonication, the obtained nanoparticles show a distribution with multiple peaks. The particle size is $69.5\pm7.3$ nm, and the PI is 0.520.

Figure 12: Example 31 PEG-PLA nanoparticle size distribution diagram, from which it can be concluded that when PEG-PLA nanoparticles are prepared under ultrasonication, the obtained nanoparticles show a distribution with single peak, the particle size is $25.0\pm0.1$ nm, and the PI is 0.152.

Figure 13: Example 32 PLGA nanoparticle size distribution diagram, from which it can be concluded that when PLGA nanoparticles are prepared under ultrasonication, the obtained nanoparticles show a distribution with single peak, the particle size is $218.5\pm2.1$ nm, and the PI is 0.063.

Figure 14: Example 33 PTX/PLGA nanoparticle size distribution diagram, from which it can be concluded that when PTX/PLGA nanoparticles are prepared under ultrasonication, the obtained nanoparticles show a distribution with single peak, the particle size is $225.9\pm1.2$ nm, and the PI is 0.027.

Figure 15: Example 34 PolyI lipid nanoparticle particle size distribution diagram, from which it can be concluded that when the PolyI lipid nanoparticle is prepared without ultrasonication, the obtained nanoparticles show a distribution with multiple peaks, the particle size is 89.6 nm, and the PI is 0.414.

Figure 16: Example 35 PolyI lipid nanoparticle particle size distribution diagram, from which it can be concluded that when the PolyI lipid nanoparticle is prepared under ultrasonication, the obtained nanoparticles show a distribution with single peak, the particle size is 85.7 nm, and the PI is 0.214.

DETAILED DESCRIPTION OF THE EMBODIMENTS

The present invention is further described below by examples, but the present invention is not limited to the scope of the described examples. Experimental methods that do not indicate specific conditions in the following examples should be selected according to conventional methods and conditions, or according to product specifications.

The endpoints of ranges and any values disclosed herein are not limited to the precise range or value, but these ranges or values are to be understood to include values close to such ranges or values. For numerical ranges, the endpoint values of each range, the endpoint values of each range and individual point values, and the individual point values can be combined with each other to obtain one or more new numerical ranges. These numerical ranges shall be deemed to be specifically disclosed herein.

Unless otherwise specified, "under ultrasonication" in the present invention refers to the simultaneous action of ultrasound and turbulent mixing process of two phases.

The abbreviations used in the present invention are shown in the table below:

| Abbreviation | Full name |
|---|---|
| D1(S) | Diameter of a spray hole in the end of the first pipeline |
| D2(IN) | Inner diameter of the second pipeline |
| D3(IN) | Inner diameter of the combined pipeline |
| Re | Reynolds number |
| Q1 | The quantity of flow of the first pipe |
| Q2 | The quantity of flow of the second pipe |
| t1 | First phase solution temperature |
| t2 | Second phase solution temperature |
| IR780 | 2-[2-[2-Chloro-3-[(1,3-dihydro-3,3-dimethyl-1-propyl-2H-indol-2-ylidene)ethylidene]-1-cyclohexen-1- yl]ethenyl]-3,3-dimethyl-1-propylindolium iodide |
| IR820 | New indocyanine green |
| ICG | Indocyanine green |
| SN-38 | 7-Ethyl-10-hydroxycamptothecin |
| deruxtecan | Deruxtecan |
| DMF | N,N-dimethylformamide |
| DMAc | N,N-dimethylacetamide |
| DMSO | Dimethyl sulfoxide |
| THF | Tetrahydrofuran |
| HMPA | Hexamethylphosphoramide |
| siRNA | Small interfering RNA |
| Ce6 | Chlorin e6 |
| DOX | Doxorubicin |
| HCPT | 10-Hydroxycamptothecin |
| CPT11 | Irinotecan |
| PTX | Paclitaxel |
| UA | Ursolic acid |
| LA | Lactobionic acid |
| PLA | Polylactic acid |
| PCL | Polycaprolactone |
| PEG-PLA | Methoxyl polyethylene glycol-polylactic acid copolymer |
| PVA | Polyvinyl alcohol |
| PolyI | Polyinosinic acid |
| ALC-0315 | Cationic lipid CAS#:2036272-55-4 |
| DSPE-PEG2000 | 1,2-Distearoyl-sn-glycero-3-phosphoethanolamine-polyethylene glycol 2000 |
| HSPC | Hydrogenated soy phosphatidylcholine |
| PEG-Phe-PLA | Methoxyl polyethylene glycol-(CO-Phenylalanine-NH)-polylactic acid copolymer |

(continued)

| Abbreviation | Full name |
|---|---|
| FNP | Flash nanoprecipitation |
| CIJM | Confined impinging jet mixer |
| MIVM | Multi-inlet vortex mixer |
| FVR | The flow velocity ratio |

The turbulent mixing part in this application is a circular pipe with a certain diameter and length, and turbulent flow conditions are achieved through one or more of the following methods:

1) Increase flow velocity:

Thorough mixing requires turbulence, Re>4000
Quantity of flow Q=πd2/4*υ

$$Re=\rho \upsilon d/\mu=4\rho Q/(\pi d\mu)>4000$$

When d=0.25 mm

$$Q> \pi\mu d=2.8 \text{ L/h}$$

When d=4 mm

$$Q > 45 \text{ L/h}$$

When d=40 mm

$$Q > 450 \text{ L/h}$$

Wherein, Re is the Reynolds number, Q is the quantity of flow, d is the pipeline diameter, υ is the fluid flow velocity in the pipe, μ is the fluid viscosity, 20oC μwater = 10-3 Pa•s
It should be pointed out that the preparation of certain composite nanoparticles of photosensitizers and anti-tumor drugs, nano-formulations with a certain particle size and particle size distribution can also be obtained with Re in the range of 500-4000.

2) Change the shape of the pipeline:

By increasing the tortuosity of the pipeline, the direction of fluid flow is forcibly changed, which enhances the fluid mixing.

3) Add a static mixer to the pipeline:

The static mixer can include but is not limited to: SV type static mixer, SX type static mixer, SL type static mixer, SH type static mixers, SK type static mixers, etc. that can divide the fluid through turbulent mixing elements, change the flow direction of the fluid, enhance the convection of the fluid, and increase the mixing of the fluid.
The SV type static mixer unit is a cylinder assembled from certain regular wave plates.
The SX type static mixer unit consists of many X-shaped units composed of crossed horizontal bars according to certain rules.

The SL type static mixer unit is composed of crossed horizontal bars according to a certain pattern to form a single X-shaped unit.

The SK type static mixer unit is composed of single-channel left and right twisted spiral plates welded together.

The SH type static mixer unit is composed of double channels, with a fluid redistribution chamber between the units.

**Example 1: Determination of drug concentration**

Instrument: Agilent1260 HPLC

Column: Waters XBridge C18 4.6*150mm, 3.5$\mu$m

Mobile phase: Use 10mmol/L sodium dihydrogen phosphate solution (adjust to pH 4.0 with phosphoric acid) as phase A, use acetonitrile as phase B, and perform gradient elution according to the following table:

| Time (min) | A% | B% |
|---|---|---|
| 0 | 80 | 20 |
| 10 | 20 | 80 |
| 11 | 80 | 20 |
| 16 | 80 | 20 |

Chromatographic parameters: flow velocity: 1ml/min; column temperature: 35°C; detection wavelength: 264 nm; injection volume: 10 $\mu$l

Calculation method: Peak area external standard method

**Example 2: Determination of nanoparticle size**

Dynamic light scattering method: The concentration of nanoparticles is 10-100 $\mu$g/ml, and the particle size and distribution of nanoparticles are measured with a nanoparticle sizer (laser light source 633 nm). Each sample is measured three times, and the average and variance of the nanoparticle size are calculated.

**Example 3: Determination of drug encapsulation efficiency**

Take 1 ml of nanoparticle solution, filter with a 0.22 $\mu$m nylon needle filter, and measure the hydrophobic drug concentration by HPLC.

$$\text{Hydrophobic drug encapsulation efficiency\%} = \frac{\text{Hydrophobic drug concentration after filtration}}{\text{Hydrophobic drug concentration before filtration}} \times 100\%$$

**Example 4: Preparation of SN-38/ICG nanoparticles (SK type static mixer, first phase 11 ml/min, second phase 193 ml/min, 20 ml produced)**

First phase solution: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.

Second phase solution: water

Spray hole diameter at the end of first pipeline D1(S)=0.6mm;

Inner diameter of the second pipeline D2(IN) =5.4mm;

Inner diameter of the combined pipeline D3(IN)=5.4mm;

Outer diameter at the end of first pipeline D1(O)=2 mm;

Outer diameter of the second pipeline D2(O)=6 mm;

Outer diameter of the combined pipeline D3(O)=6 mm;

Combined phase length=90 mm;

First phase solution temperature t1=25°C;

Second phase solution temperature t2=25°C;

| First phase ml/min | Second phase ml/min |
|---|---|
| 11 | 193 |

Calculated based on the fluid in the circular pipeline, the combined phase Re=747, with SK type static mixer, static mixer size: 5.3mm*85 mm, with a total of 16 repeating spiral plates.
The first phase outlet flow velocity is: 0.648 m/s
The combined phase flow velocity is: 0.148 m/s
FVR=4.4
Production quantity: 20 ml

| | Ultra sound | D10 (nm) | D50 (nm) | D90 (nm) | Average diameter (nm) | PDI | Filtration encapsulation efficiency (SN-38) |
|---|---|---|---|---|---|---|---|
| (1) | Y* | 96±4 | 115±7 | 142±13 | 98±8 | 0.31±0.19 | 94.8% |
| (2) | N | 88±16 | 231±85 | 413±34 | 167±7 | 0.45±0.05 | 53.6% |

*Under the simultaneous action of ultrasound and turbulent mixing, the two phases were combined in a 200W ultrasonic bath.

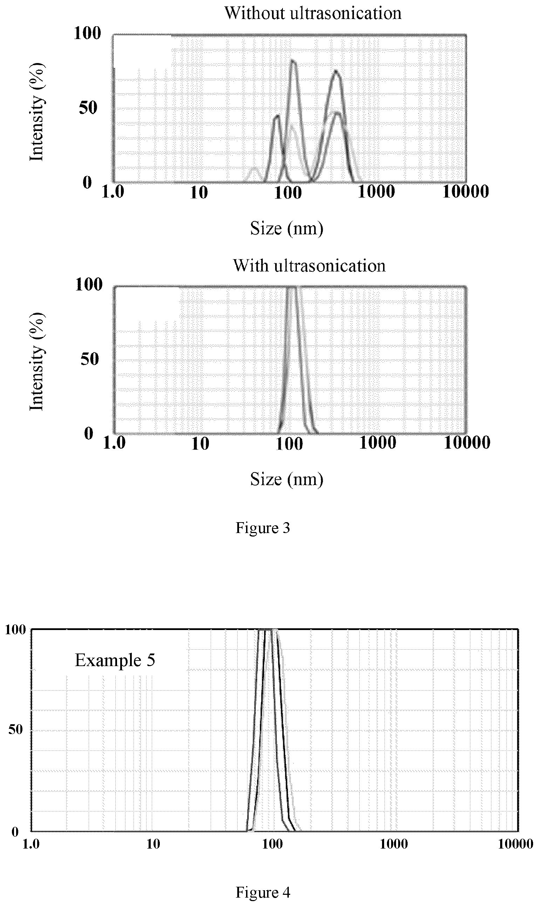

By comparing (1) and (2) above, the encapsulation efficiency of SN-38 increased from 53.5% to 94.8%, the particle size distribution changed from multiple peaks to single peak (Figure 3), and the average particle size decreased significantly.

**Example 1: Preparation of SN-38/ICG nanoparticles (first phase 60 ml/min, second phase 936 ml/min, without ultrasonication, 20 ml produced)**

First phase solution: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
Second phase solution: water
Spray hole diameter at the end of first pipeline D1(S)=0.6mm;
Inner diameter of the second pipeline D2(IN) =5.4mm;
Inner diameter of the combined pipeline D3(IN)=5.4mm;
Outer diameter at the end of first pipeline D1(O)=2 mm;
Outer diameter of the second pipeline D2(O)=6 mm;
Outer diameter of the combined pipeline D3(O)=6 mm;
Combined phase length=90 mm;
First phase solution temperature t1=25°C;
Second phase solution temperature t2=25°C;

| First phase ml/min | Second phase ml/min |
|---|---|
| 60 | 936 |

Calculated based on the fluid in the circular pipeline, the combined phase Re=3868, with no static mixer.
The first phase outlet flow velocity is: 3.54m/s
The second phase flow velocity is: 0.68m/s
FVR=5.2
Production quantity: 20 ml

| D10 nm | D50 nm | D90 nm | Average diameter nm | D90/D10 | Encapsulation efficiency (SN-38) |
|---|---|---|---|---|---|
| 79±7 | 96±8 | 124±13 | 98±4 | 1.57 | 98.4 % |

**Example 2: Preparation of SN-38/ICG nanoparticles (first phase 60 ml/min, second phase 936 ml/min, without ultrasonication, 1.0 L produced)**

First phase solution: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
Second phase solution: water
Spray hole diameter at the end of first pipeline Di(S)=0.6mm;
Inner diameter of the second pipeline $D_2$(IN) =5.4mm;
Inner diameter of the combined pipeline $D_3$(IN)=5.4mm;
Outer diameter at the end of first pipeline $D_1$(O)=2 mm;
Outer diameter of the second pipeline $D_2$(O)=6 mm;
Outer diameter of the combined pipeline $D_3$(O)=6 mm;
Combined phase length=360 mm;
First phase solution temperature $t_1$=25°C;
Second phase solution temperature $t_2$=25°C;

| First phase ml/min | Second phase ml/min |
|---|---|
| 60 | 936 |

Calculated based on the fluid in the circular pipeline, the combined phase Re=3868, with no static mixer.
The first phase outlet flow velocity is: 3.54m/s
The second phase flow velocity is: 0.68m/s
FVR=5.2
Production quantity: 1.0 L

| D10 nm | D50 nm | D90 nm | Average diameter nm | D90/D10 | Encapsulation efficiency (SN-38) |
|---|---|---|---|---|---|
| 82±3 | 115±6 | 196±3 | 111±6 | 2.39 | 92.4 % |

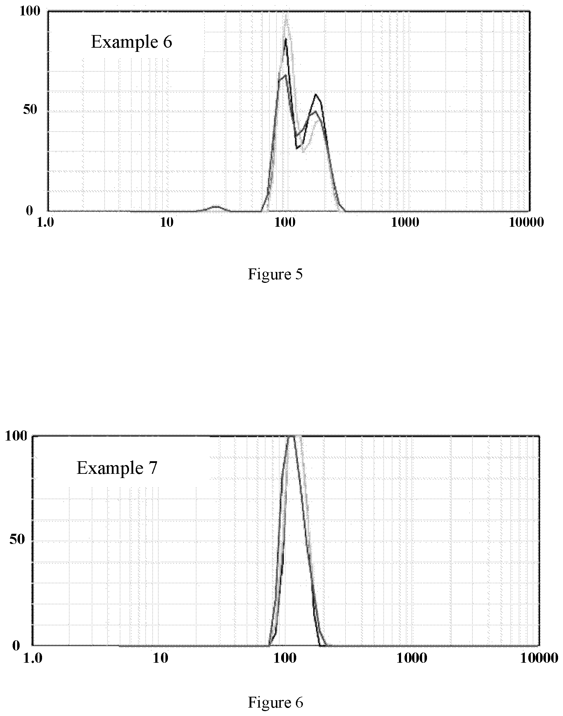

Comparing examples 5 and 6, for the scale-up preparation of nanoparticles (from 20 mL to 1000 mL), the encapsulation efficiency decreases significantly, the ratio of D90 to D10 increases significantly, and the particle size shows a distribution with multiple peaks without ultrasonication (Figure 5).

**Example 3: Preparation of SN-38/ICG nanoparticles (first phase 60 ml/min, second phase 936 ml/min, under ultrasonication, 1.0 L produced)**

First phase solution: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
Second phase solution: water
Spray hole diameter at the end of first pipeline Di(S)=0.6mm;
Inner diameter of the second pipeline $D_2$(IN) =5.4mm;
Inner diameter of the combined pipeline $D_3$(IN)=5.4mm;
Outer diameter at the end of first pipeline $D_1$(O)=2 mm;
Outer diameter of the second pipeline $D_2$(O)=6 mm;
Outer diameter of the combined pipeline $D_3$(O)=6 mm;
Combined phase length=360 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 60 | 936 |

Calculated based on the fluid in the circular pipeline, the combined phase Re=3868, with no static mixer.
The first phase outlet flow velocity is: 3.54m/s

The second phase flow velocity is: 0.68m/s
FVR=5.2
Production quantity: 1.0 L

| D10 nm | D50 nm | D90 nm | Average diameter nm | D90/D10 | Encapsulation efficiency (SN-38) |
|---|---|---|---|---|---|
| 89±10 | 100±12 | 145±16 | 109±10 | 1.63 | 97.5 % |

Comparing examples 7 and 6, for the scale-up preparation of nanoparticles under ultrasonication, the encapsulation efficiency is significantly increased (97.5% vs 92.4%), the ratio of D90 to D10 is reduced (1.63 vs 2.39), and the particle size shows a distribution with single peak (Figure 6). Under ultrasonication, the scale-up preparation of nanoparticles is similar to the experimental results of the small amount preparation conditions (Example 5).

**Example 4: Preparation of SN-38/ICG nanoparticles (SK type static mixer, first phase 80 ml/min, second phase 1248 ml/min, 20 ml produced)**

First phase solution: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
Second phase solution: water
Spray hole diameter at the end of first pipeline $D_i(S)$=0.6mm;
Inner diameter of the second pipeline $D_2(IN)$ =5.4mm;
Inner diameter of the combined pipeline $D_3(IN)$=5.4mm;
Outer diameter at the end of first pipeline $D_1(O)$=2 mm;
Outer diameter of the second pipeline $D_2(O)$=6 mm;
Outer diameter of the combined pipeline $D_3(O)$=6 mm;
Combined phase length=90 mm;
First phase solution temperature ti=25°C;
Second phase solution temperature $t_2$=25°C;

| First phase ml/min | Second phase ml/min |
|---|---|
| 80 | 1248 |

Calculated based on the fluid in the circular pipeline, the combined phase Re=5158, with SK type static mixer, static mixer size: 5.3mm*85 mm, with a total of 16 repeating spiral plates.
The first phase outlet flow velocity is: 4.72m/s
The second phase flow velocity is: 0.91m/s
FVR=5.2
Production quantity: 20 ml

| D10 nm | D50 nm | D90 nm | Average diameter nm | D90/D10 | Encapsulation efficiency (SN-38) |
|---|---|---|---|---|---|
| 83±17 | 101±23 | 126±35 | 109±5 | 1.52 | 98.8 % |

**Example 5: Preparation of SN-38/ICG nanoparticles (SK type static mixer, first phase 80 ml/min, second phase 1248 ml/min, without ultrasonication, 425 ml produced)**

First phase solution: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
Second phase solution: water
Spray hole diameter at the end of first pipeline $D_1(S)$=0.6mm;
Inner diameter of the second pipeline $D_2(IN)$ =5.4mm;
Inner diameter of the combined pipeline $D_3(IN)$=5.4mm;
Outer diameter at the end of first pipeline $D_1(O)$=2 mm;
Outer diameter of the second pipeline $D_2(O)$=6 mm;
Outer diameter of the combined pipeline $D_3(O)$=6 mm;

Combined phase length=90 mm;
First phase solution temperature $t_1$=25°C;
Second phase solution temperature $t_2$=25°C;

| First phase ml/min | Second phase ml/min |
|---|---|
| 80 | 1248 |

Calculated based on the fluid in the circular pipeline, the combined phase Re=5158, with SK type static mixer, static mixer size: 5.3mm*85 mm, with a total of 16 repeating spiral plates.
The first phase outlet flow velocity is: 4.72m/s
The second phase flow velocity is: 0.91m/s
FVR=5.2
Production quantity: 425 ml

| D10 nm | D50 nm | D90 nm | Average diameter nm | D90/D10 | Encapsulation efficiency (SN-38) |
|---|---|---|---|---|---|
| 93±3 | 121±1 | 180±10 | 126±4 | 1.94 | 92.2 % |

Comparing Examples 8 and 9, for the scale-up preparation of nanoparticles without ultrasonication (from 20mL to 425mL), the encapsulation efficiency decreases significantly and the ratio of D90 to D10 increases.

**Example 6: Preparation of SN-38/ICG nanoparticles (SK type static mixer, first phase 80 ml/min, second phase 1248 ml/min, under ultrasonication, 425 ml produced)**

First phase solution: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
Second phase solution: water
Spray hole diameter at the end of first pipeline Di(S)=0.6mm;
Inner diameter of the second pipeline $D_2$(IN) =5.4mm;
Inner diameter of the combined pipeline $D_3$(IN)=5.4mm;
Outer diameter at the end of first pipeline $D_1$(O)=2 mm;
Outer diameter of the second pipeline $D_2$(O)=6 mm;
Outer diameter of the combined pipeline $D_3$(O)=6 mm;
Combined phase length=90 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 80 | 1248 |

Calculated based on the fluid in the circular pipeline, the combined phase Re=5158, with SK type static mixer, static mixer size: 5.3mm*85 mm, with a total of 16 repeating spiral plates.
The first phase outlet flow velocity is: 4.72m/s
The second phase flow velocity is: 0.91m/s
FVR=5.2
Production quantity: 425 ml

| D10 nm | D50 nm | D90 nm | Average diameter nm | D90/D10 | Encapsulation efficiency (SN-38) |
|---|---|---|---|---|---|
| 96±1 | 116±4 | 146±10 | 113±4 | 1.52 | 97.0 % |

Comparing examples 10 and 9, for the scale-up preparation of nanoparticles under ultrasonication, the encapsulation efficiency is significantly increased (97.0% vs 92.2%), the ratio of D90 to D10 is reduced (1.52 vs 1.94), and the particle size shows a distribution with single peak. Under ultrasonication, the scale-up preparation of nanoparticles is similar to the experimental results of the small amount preparation conditions (Example 8).

**Example 7: Preparation of PTX/ICG nanoparticles (first phase 60 ml/min, second phase 890 ml/min, without ultrasonication, 20 mL produced)**

First phase solution: PTX and ICG were co-dissolved in methanol, PTX concentration: 100 mg/ml, ICG concentration: 50 mg/ml, filtration with 0.22 $\mu$m nylon filter membrane.
Second phase solution: water
Spray hole diameter at the end of first pipeline Di(S)=0.4 mm;
Inner diameter of the second pipeline $D_2$(IN) =3.0 mm;
Inner diameter of the combined pipeline $D_3$(IN)=3.0 mm;
Outer diameter at the end of first pipeline $D_1$(O)=1.0 mm;
Combined phase length=90 mm;
First phase solution temperature $t_1$=25°C;
Second phase solution temperature $t_2$=25°C;

| First phase ml/min | Second phase ml/min |
| --- | --- |
| 60 | 890 |

Calculated based on the fluid in the circular pipeline, the combined phase Re=6623.
The first phase outlet flow velocity is: 7.58 m/s
The second phase flow velocity is: 2.24 m/s
FVR=3.38
Production quantity: 20 ml

| D10 nm | D50 nm | D90 nm | Average diameter nm | D90/D10 | Encapsulation efficiency (PTX) |
| --- | --- | --- | --- | --- | --- |
| 68±3 | 79±4 | 90±9 | 75±3 | 1.32 | 98.2 % |

**Example 8: Preparation of PTX/ICG nanoparticles (first phase 60 ml/min, second phase 890 ml/min, without ultrasonication, 400 mL produced)**

First phase solution: PTX and ICG were co-dissolved in methanol, PTX concentration: 100 mg/ml, ICG concentration: 50 mg/ml, filtration with 0.22 $\mu$m nylon filter membrane.
Second phase solution: water
Spray hole diameter at the end of first pipeline Di(S)=0.4 mm;
Inner diameter of the second pipeline $D_2$(IN) =3.0 mm;
Inner diameter of the combined pipeline $D_3$(IN)=3.0 mm;
Outer diameter at the end of first pipeline $D_1$(O)=1.0 mm;
Combined phase length=90 mm;
First phase solution temperature $t_1$=25°C;
Second phase solution temperature $t_2$=25°C;

| First phase ml/min | Second phase ml/min |
| --- | --- |
| 60 | 890 |

Calculated based on the fluid in the circular pipeline, the combined phase Re=6623.
The first phase outlet flow velocity is: 7.58 m/s
The second phase flow velocity is: 2.24 m/s
FVR=3.38
Production quantity: 400 ml

| D10 nm | D50 nm | D90 nm | Average diameter nm | D90/D10 | Encapsulation efficiency (PTX) |
| --- | --- | --- | --- | --- | --- |
| 64±7 | 82±5 | 102±13 | 81±5 | 1.59 | 93.4 % |

Comparing Examples 11 and 12, for the scale-up preparation of nanoparticles without ultrasonication (from 20 mL to 400 mL), the encapsulation efficiency decreases significantly and the ratio of D90 to D10 increases.

**Example 9: Preparation of PTX/ICG nanoparticles (first phase 60 ml/min, second phase 890 ml/min, under ultrasonication, 400 mL produced)**

First phase solution: PTX and ICG were co-dissolved in methanol, PTX concentration: 100 mg/ml, ICG concentration: 50 mg/ml, filtration with 0.22 $\mu$m nylon filter membrane.
Second phase solution: water
Spray hole diameter at the end of first pipeline Di(S)=0.4 mm;
Inner diameter of the second pipeline $D_2$(IN) =3.0 mm;
Inner diameter of the combined pipeline $D_3$(IN)=3.0 mm;
Outer diameter at the end of first pipeline $D_1$(O)=1.0 mm;
Combined phase length=90 mm;
First phase solution temperature $t_1$=25°C;
Second phase solution temperature $t_2$=25°C;
Two phases combine in a 200W ultrasonic bath

| First phase ml/min | Second phase ml/min |
| --- | --- |
| 60 | 890 |

Calculated based on the fluid in the circular pipeline, the combined phase Re=6623.
The first phase outlet flow velocity is: 7.58 m/s
The second phase flow velocity is: 2.24 m/s
FVR=3.38
Production quantity: 400 ml

| D10 nm | D50 nm | D90 nm | Average diameter nm | D90/D10 | Encapsulation efficiency (PTX) |
| --- | --- | --- | --- | --- | --- |
| 70±3 | 74±2 | 89±10 | 72±2 | 1.27 | 98.7 % |

Comparing examples 13 and 12, for the scale-up preparation of nanoparticles under ultrasonication, the encapsulation efficiency is significantly increased (98.7% vs 93.4%), the ratio of D90 to D10 is reduced (1.27 vs 1.59), and the particle size shows a distribution with single peak. Under ultrasonication, the scale-up preparation of nanoparticles is similar to the experimental results of the small amount preparation conditions (Example 11).

**Example 10: Preparation of Curcumin/CPT11 nanoparticles (first phase 60 ml/min, second phase 890 ml/min, without ultrasonication, 20 mL produced)**

First phase solution: curcumin and CPT11 were co-dissolved in DMSO, curcumin concentration: 50 mg/ml, CPT11 concentration: 150 mg/ml, filtration with 0.22 $\mu$m nylon filter membrane.
Second phase solution: water
Spray hole diameter at the end of first pipeline $D_1$(S)=0.4 mm;
Inner diameter of the second pipeline $D_2$(IN) =3.0 mm;
Inner diameter of the combined pipeline $D_3$(IN)=3.0 mm;
Outer diameter at the end of first pipeline $D_1$(O)=1.0 mm;
Combined phase length=90 mm;
First phase solution temperature $t_1$=25°C;
Second phase solution temperature $t_2$=25°C;

| First phase ml/min | Second phase ml/min |
| --- | --- |
| 60 | 890 |

Calculated based on the fluid in the circular pipeline, the combined phase Re=6623.
The first phase outlet flow velocity is: 7.58 m/s

The second phase flow velocity is: 2.24 m/s
FVR=3.38
Production quantity: 20 ml

| D10 nm | D50 nm | D90 nm | Average diameter nm | D90/D10 | Encapsulation efficiency (curcumin) |
|---|---|---|---|---|---|
| 80±3 | 93±2 | 113±1 | 96±2 | 1.41 | 97.4 % |

**Example 11: Preparation of Curcumin/CPT11 nanoparticles (first phase 60 ml/min, second phase 890 ml/min, without ultrasonication, 400 mL produced)**

First phase solution: curcumin and CPT11 were co-dissolved in DMSO, curcumin concentration: 50 mg/ml, CPT11 concentration: 150 mg/ml, filtration with 0.22 $\mu$m nylon filter membrane.
Second phase solution: water
Spray hole diameter at the end of first pipeline Di(S)=0.4 mm;
Inner diameter of the second pipeline $D_2(IN)$ =3.0 mm;
Inner diameter of the combined pipeline $D_3(IN)$=3.0 mm;
Outer diameter at the end of first pipeline $D_1(O)$=1.0 mm;
Combined phase length=90 mm;
First phase solution temperature $t_1$=25°C;
Second phase solution temperature $t_2$=25°C;

| First phase ml/min | Second phase ml/min |
|---|---|
| 60 | 890 |

Calculated based on the fluid in the circular pipeline, the combined phase Re=6623.
The first phase outlet flow velocity is: 7.58 m/s
The second phase flow velocity is: 2.24 m/s
FVR=3.38
Production quantity: 400 ml

| D10 nm | D50 nm | D90 nm | Average diameter nm | D90/D10 | Encapsulation efficiency (curcumin) |
|---|---|---|---|---|---|
| 83±5 | 96±7 | 149±15 | 103±13 | 1.80 | 93.6 % |

Comparing Examples 15 and 14, for the scale-up preparation of nanoparticles without ultrasonication (from 20 mL to 400 mL), the encapsulation efficiency decreases significantly and the ratio of D90 to D10 increases.

**Example 12: Preparation of Curcumin/CPT11 nanoparticles (first phase 60 ml/min, second phase 890 ml/min, under ultrasonication, 400 mL produced)**

First phase solution: curcumin and CPT11 were co-dissolved in DMSO, curcumin concentration: 50 mg/ml, CPT11 concentration: 150 mg/ml, filtration with 0.22 $\mu$m nylon filter membrane.
Second phase solution: water
Spray hole diameter at the end of first pipeline Di(S)=0.4 mm;
Inner diameter of the second pipeline $D_2(IN)$ =3.0 mm;
Inner diameter of the combined pipeline $D_3(IN)$=3.0 mm;
Outer diameter at the end of first pipeline $D_1(O)$=1.0 mm;
Combined phase length=90 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 60 | 890 |

Calculated based on the fluid in the circular pipeline, the combined phase Re=6623.
The first phase outlet flow velocity is: 7.58 m/s
The second phase flow velocity is: 2.24 m/s
FVR=3.38
Production quantity: 400 ml

| D10 nm | D50 nm | D90 nm | Average diameter nm | D90/D10 | Encapsulation efficiency (curcumin) |
|---|---|---|---|---|---|
| 75±2 | 87±3 | 105±4 | 91±4 | 1.40 | 98.2 % |

Comparing Examples 16 and 15, for the scale-up preparation of nanoparticles under ultrasonication, the encapsulation efficiency is significantly increased (98.2% vs 93.6%), the ratio of D90 to D10 is reduced (1.40 vs 1.80), and the particle size shows a distribution with single peak. Under ultrasonication, the scale-up preparation of nanoparticles is similar to the experimental results of the small amount preparation conditions (Example 14).

**Example 13: Preparation of SN-38/CPT11 nanoparticles (first phase 60 ml/min, second phase 890 ml/min, without ultrasonication, 20 mL produced)**

First phase solution: SN-38 and CPT11 were co-dissolved in DMSO, SN-38 concentration: 40 mg/ml, CPT11 concentration: 40 mg/ml, filtration with 0.22 $\mu$m nylon filter membrane.
Second phase solution: water
Spray hole diameter at the end of first pipeline Di(S)=0.4 mm;
Inner diameter of the second pipeline $D_2$(IN) =3.0 mm;
Inner diameter of the combined pipeline $D_3$(IN)=3.0 mm;
Outer diameter at the end of first pipeline $D_1$(O)=1.0 mm;
Combined phase length=90 mm;
First phase solution temperature $t_1$=25°C;
Second phase solution temperature $t_2$=25°C;

| First phase ml/min | Second phase ml/min |
|---|---|
| 60 | 890 |

Calculated based on the fluid in the circular pipeline, the combined phase Re=6623.
The first phase outlet flow velocity is: 7.58 m/s
The second phase flow velocity is: 2.24 m/s
FVR=3.38
Production quantity: 20 ml

| D10 nm | D50 nm | D90 nm | Average diameter nm | D90/D10 | Encapsulation efficiency (SN-38) |
|---|---|---|---|---|---|
| 89±6 | 95±3 | 109±4 | 93±2 | 1.22 | 97.8 % |

**Example 14: Preparation of SN-38/CPT11 nanoparticles (first phase 60 ml/min, second phase 890 ml/min, without ultrasonication, 400 mL produced)**

First phase solution: SN-38 and CPT11 were co-dissolved in DMSO, SN-38 concentration: 40 mg/ml, CPT11 concentration: 40 mg/ml, filtration with 0.22 $\mu$m nylon filter membrane.
Second phase solution: water
Spray hole diameter at the end of first pipeline Di(S)=0.4 mm;
Inner diameter of the second pipeline $D_2$(IN) =3.0 mm;
Inner diameter of the combined pipeline $D_3$(IN)=3.0 mm;
Outer diameter at the end of first pipeline $D_1$(O)=1.0 mm;
Combined phase length=90 mm;
First phase solution temperature ti=25°C;
Second phase solution temperature $t_2$=25°C;

| First phase ml/min | Second phase ml/min |
|---|---|
| 60 | 890 |

Calculated based on the fluid in the circular pipeline, the combined phase Re=6623.
The first phase outlet flow velocity is: 7.58 m/s
The second phase flow velocity is: 2.24 m/s
FVR=3.38
Production quantity: 400 ml

| D10 nm | D50 nm | D90 nm | Average diameter nm | D90/D10 | Encapsulation efficiency (SN-38) |
|---|---|---|---|---|---|
| 76±5 | 108±7 | 154±16 | 97±5 | 2.02 | 90.2 % |

Comparing Examples 17 and 18, for the scale-up preparation of nanoparticles without ultrasonication (from 20 mL to 400 mL), the encapsulation efficiency decreases significantly and the ratio of D90 to D10 increases.

**Example 15: Preparation of SN-38/CPT11 nanoparticles (first phase 60 ml/min, second phase 890 ml/min, under ultrasonication, 400 mL produced)**

First phase solution: SN-38 and CPT11 were co-dissolved in DMSO, SN-38 concentration: 40 mg/ml, CPT11 concentration: 40 mg/ml, filtration with 0.22 $\mu$m nylon filter membrane.
Second phase solution: water
Spray hole diameter at the end of first pipeline Di(S)=0.4 mm;
Inner diameter of the second pipeline $D_2$(IN) =3.0 mm;
Inner diameter of the combined pipeline $D_3$(IN)=3.0 mm;
Outer diameter at the end of first pipeline $D_1$(O)=1.0 mm;
Combined phase length=90 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 60 | 890 |

Calculated based on the fluid in the circular pipeline, the combined phase Re=6623.
The first phase outlet flow velocity is: 7.58 m/s
The second phase flow velocity is: 2.24 m/s
FVR=3.38
Production quantity: 400 ml

| D10 nm | D50 nm | D90 nm | Average diameter nm | D90/D10 | Encapsulation efficiency (SN-38) |
|---|---|---|---|---|---|
| 92±3 | 97±4 | 107±2 | 94±3 | 1.16 | 98.1 % |

Comparing Examples 19 and 18, for the scale-up preparation of nanoparticles under ultrasonication, the encapsulation efficiency is significantly increased (98.1% vs 90.2%), the ratio of D90 to D10 is reduced (1.16 vs 2.02), and the particle size shows a distribution with single peak. Under ultrasonication, the scale-up preparation of nanoparticles is similar to the experimental results of the small amount preparation conditions (Example 17).

**Example 16: Preparation of HSPC/CHOL/DSPE-PEG blank liposome (first phase 10 ml/min, second phase 100 ml/min, without ultrasonication, 50 mL produced)**

First phase solution: HSPC, CHOL, DSPE-PEG$_{2k}$ were co-dissolved in ethanol and filtered with 0.22 $\mu$m nylon

filter membrane. HSPC: CHOL: DSPE-PEG$_{2k}$=56:38:5 (molar ratio), HSPC concentration: 10 mg/ml.

Second phase solution: 120 mM ammonium sulfate in water.

Spray hole diameter at the end of first pipeline Di(S)=0.25 mm;

Inner diameter of the second pipeline D$_2$(IN) =0.8 mm;

Inner diameter of the combined pipeline D$_3$(IN)=0.8 mm;

Outer diameter at the end of first pipeline D$_1$(O)=0.35 mm;

Combined phase length=360 mm;

First phase solution temperature t$_1$=25°C;

Second phase solution temperature t$_2$=25°C;

| First phase ml/min | Second phase ml/min |
|---|---|
| 10 | 100 |

Calculated based on the fluid in the circular pipeline, the combined phase Re=2884.

The first phase outlet flow velocity is: 3.4 m/s

The second phase flow velocity is: 3.65 m/s

FVR=0.93

Production quantity: 50 ml

| D10 nm | D50 nm | D90 nm | Average diameter nm | D90/D10 | Encapsulation efficiency |
|---|---|---|---|---|---|
| 53±57 | 162±32 | 208±63 | - | 3.92 | - |

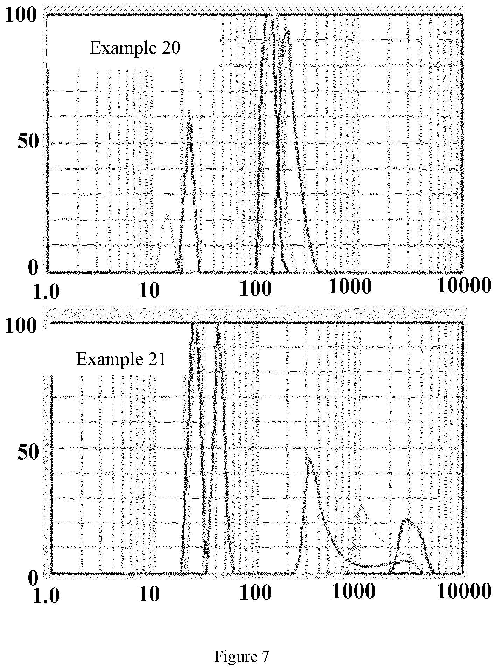

The particle size of blank liposomes prepared without ultrasonication showed a distribution with multiple peaks (Figure 7), and there were large particle size distributions and variances in blank liposomes.

**Example 17: Preparation of HSPC/CHOL/DSPE-PEG blank liposome (first phase 10 ml/min, second phase 200 ml/min, without ultrasonication, 50 mL produced)**

First phase solution: HSPC, CHOL, DSPE-PEG$_{2k}$ were co-dissolved in ethanol and filtered with 0.22 $\mu$m nylon filter membrane. HSPC: CHOL: DSPE-PEG$_{2k}$=56:38:5 (molar ratio), HSPC concentration: 10 mg/ml.

Second phase solution: 120 mM ammonium sulfate in water.

Spray hole diameter at the end of first pipeline Di(S)=0.25 mm;

Inner diameter of the second pipeline D$_2$(IN) =0.8 mm;

Inner diameter of the combined pipeline D$_3$(IN)=0.8 mm;

Outer diameter at the end of first pipeline D$_1$(O)=0.35 mm;

Combined phase length=360 mm;

First phase solution temperature t$_1$=25°C;

Second phase solution temperature t$_2$=25°C;

| First phase ml/min | Second phase ml/min |
|---|---|
| 10 | 200 |

Calculated based on the fluid in the circular pipeline, the combined phase Re=5505.

The first phase outlet flow velocity is: 3.40 m/s

The second phase flow velocity is: 6.96 m/s

FVR=0.49

Production quantity: 50 ml

| D10 nm | D50 nm | D90 nm | Average diameter nm | D90/D10 | Encapsulation efficiency |
|---|---|---|---|---|---|
| 29±10 | 103±131 | 1931±1226 | | 66.5 | - |

The particle size of blank liposomes prepared without ultrasonication showed a distribution with multiple peaks (Figure 7), and there were large particle size distributions and variances in blank liposomes.

**Example 18: Preparation of HSPC/CHOL/DSPE-PEG blank liposome (first phase 10 ml/min, second phase 100 ml/min, under ultrasonication, 50 mL produced)**

First phase solution: HSPC, CHOL, DSPE-PEG$_{2k}$ were co-dissolved in ethanol and filtered with 0.22 $\mu$m nylon filter membrane. HSPC: CHOL: DSPE-PEG$_{2k}$=56:38:5 (molar ratio), HSPC concentration: 10 mg/ml.

Second phase solution: 120 mM ammonium sulfate in water.

Spray hole diameter at the end of first pipeline Di(S)=0.25 mm;

Inner diameter of the second pipeline $D_2$(IN) =0.8 mm;

Inner diameter of the combined pipeline $D_3$(IN)=0.8 mm;

Outer diameter at the end of first pipeline $D_1$(O)=0.35 mm;

Combined phase length=360 mm;

First phase solution temperature $t_1$=25°C;

Second phase solution temperature $t_2$=25°C;

Two phases combine in a 200W ultrasonic bath

| First phase ml/min | Second phase ml/min |
| --- | --- |
| 10 | 100 |

Calculated based on the fluid in the circular pipeline, the combined phase Re=2884.

The first phase outlet flow velocity is: 3.4 m/s

The second phase flow velocity is: 3.65 m/s

FVR=0.93

Production quantity: 50 ml

| D10 nm | D50 nm | D90 nm | Average diameter nm | D90/D10 |
| --- | --- | --- | --- | --- |
| 107.3± 2.1 | 123.7± 3.2 | 144.7± 6.7 | 131.7± 2.9 | 1.35 |

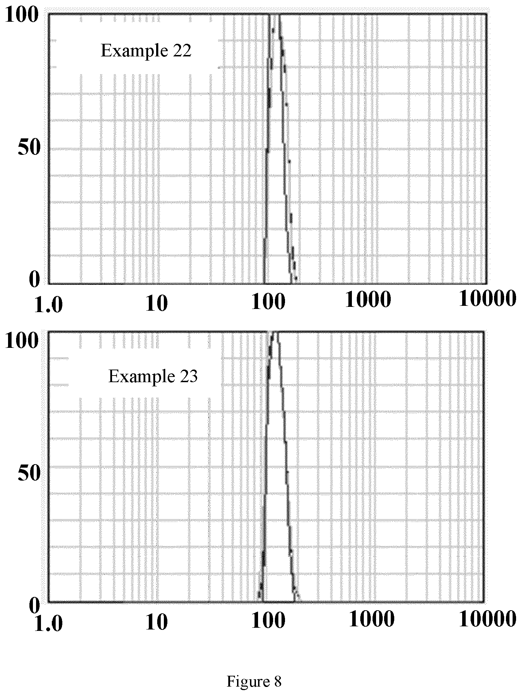

Comparing Examples 22 and 20, for the preparation of blank liposomes under ultrasonication, the particle size shows a distribution with single peak (Figure 8).

**Example 19: Preparation of HSPC/CHOL/DSPE-PEG blank liposome (first phase 10 ml/min, second phase 200 ml/min, under ultrasonication, 50 mL produced)**

First phase solution: HSPC, CHOL, DSPE-PEG$_{2k}$ were co-dissolved in ethanol and filtered with 0.22 $\mu$m nylon filter membrane. HSPC: CHOL: DSPE-PEG$_{2k}$=56:38:5 (molar ratio), HSPC concentration: 10 mg/ml.

Second phase solution: 120 mM ammonium sulfate in water.

Spray hole diameter at the end of first pipeline Di(S)=0.25 mm;

Inner diameter of the second pipeline $D_2$(IN) =0.8 mm;

Inner diameter of the combined pipeline $D_3$(IN)=0.8 mm;

Outer diameter at the end of first pipeline $D_1$(O)=0.35 mm;

Combined phase length=360 mm;

First phase solution temperature $t_1$=25°C;

Second phase solution temperature $t_2$=25°C;

Two phases combine in a 200W ultrasonic bath

| First phase ml/min | Second phase ml/min |
| --- | --- |
| 10 | 200 |

Calculated based on the fluid in the circular pipeline, the combined phase Re=5505.

The first phase outlet flow velocity is: 3.40 m/s

The second phase flow velocity is: 6.96 m/s

FVR=0.49

Production quantity: 50 ml

| D10 nm | D50 nm | D90 nm | Average diameter nm | D90/D10 |
|---|---|---|---|---|
| 99.5± 0.9 | 115.3± 1.1 | 138.3± 1.8 | 115.3± 1.6 | 1.39 |

Comparing Examples 23 and 22, for the preparation of blank liposomes under ultrasonication, when the flow velocity of the second phase increases, the average diameter of the blank liposomes decreases. The particle size shows a distribution with single peak (Figure 8).

**Example 24: Preparation of PTX/HSPC/CHOL/DSPE-PEG liposome (first phase 10 ml/min, second phase 200 ml/min, under ultrasonication, 50 mL produced)**

First phase solution: PTX, HSPC, CHOL, DSPE-PEG$_{2k}$ were co-dissolved in ethanol and filtered with 0.22 $\mu$m nylon filter membrane. PTX: HSPC: CHOL: DSPE-PEG$_{2k}$=9:89:57:4 (molar ratio), HSPC concentration: 10 mg/ml.
Second phase solution: water.
Spray hole diameter at the end of first pipeline Di(S)=0.25 mm;
Inner diameter of the second pipeline $D_2$(IN) =0.8 mm;
Inner diameter of the combined pipeline $D_3$(IN)=0.8 mm;
Outer diameter at the end of first pipeline $D_1$(O)=0.35 mm;
Combined phase length=360 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 10 | 200 |

Calculated based on the fluid in the circular pipeline, the combined phase Re=5505.
The first phase outlet flow velocity is: 3.40 m/s
The second phase flow velocity is: 6.96 m/s
FVR=0.49
Production quantity: 50 ml

| D10 nm | D50 nm | D90 nm | Average diameter nm | D90/D10 | Encapsulation efficiency |
|---|---|---|---|---|---|
| 45.9±1.7 | 87.3±4.3 | 178.8±10.9 | 81.1±1.7 | 3.89 | 82.1% |

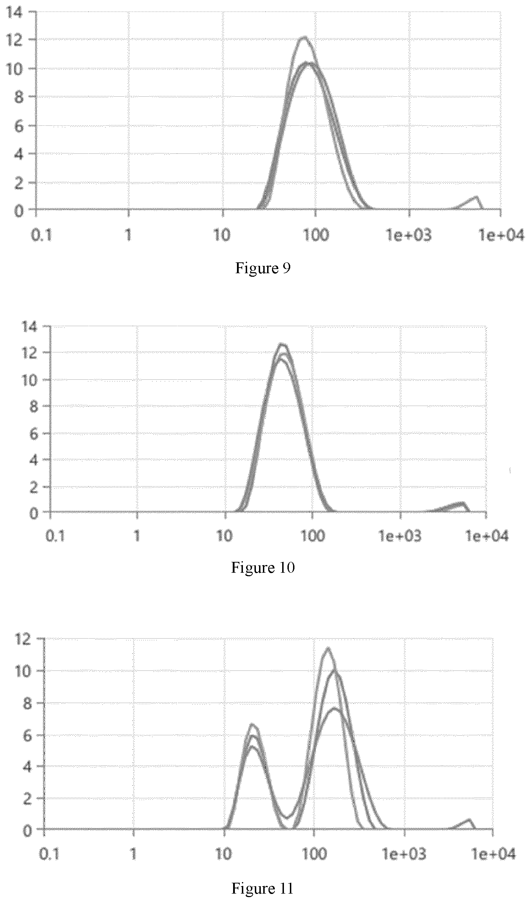

Under ultrasonication , PTX liposomes with smaller particle sizes can be prepared, and the particle size shows a distribution with single peak (Figure 9).

**Example 25: Preparation of PTX/PEG-PLA polymeric micelle (first phase 14 ml/min, second phase 210 ml/min, without ultrasonication, 50 mL produced)**

First phase solution: PTX, PEG-PLA were co-dissolved in acetonitrile, PEG-PLA (50 mg/mL), PTX (15 mg/mL), filtration with 0.22 $\mu$m nylon filter membrane.
Second phase solution: water.
Spray hole diameter at the end of first pipeline Di(S)=0.25 mm;
Inner diameter of the second pipeline $D_2$(IN) =0.8 mm;
Inner diameter of the combined pipeline $D_3$(IN)=0.8 mm;
Outer diameter at the end of first pipeline $D_1$(O)=0.35 mm;
Combined phase length=360 mm;
First phase solution temperature $t_1$=25°C;
Second phase solution temperature $t_2$=25°C;

| First phase ml/min | Second phase ml/min |
|---|---|
| 14 | 210 |

Calculated based on the fluid in the circular pipeline, the combined phase Re=5872.
The first phase outlet flow velocity is: 4.75 m/s
The second phase flow velocity is: 7.43 m/s
FVR=0.64
Production quantity: 50 ml
The solution obtained without ultrasonication had more white flocculent precipitate, and the particle size could not be detected.

**Example 26: Preparation of PTX/PEG-PLA polymeric micelle (first phase 14 ml/min, second phase 210 ml/min, under ultrasonication, 50 mL produced)**

First phase solution: PTX, PEG-PLA were co-dissolved in acetonitrile, PEG-PLA (50 mg/mL), PTX (15 mg/mL), filtration with 0.22 $\mu$m nylon filter membrane.
Second phase solution: water.
Spray hole diameter at the end of first pipeline Di(S)=0.25 mm;
Inner diameter of the second pipeline $D_2$(IN) =0.8 mm;
Inner diameter of the combined pipeline $D_3$(IN)=0.8 mm;
Outer diameter at the end of first pipeline $D_1$(O)=0.35 mm;
Combined phase length=360 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 14 | 210 |

Calculated based on the fluid in the circular pipeline, the combined phase Re=5872.
The first phase outlet flow velocity is: 4.75 m/s
The second phase flow velocity is: 7.43 m/s
FVR=0.64
Production quantity: 50 ml

| | D10 (nm) | D50 (nm) | D90 (nm) | Average diameter (nm) | PDI | Encapsulation efficiency |
|---|---|---|---|---|---|---|
| 1 | 20.4 | 23.3 | 26.6 | 24.9 | 0.07 | 85.6% |
| 2 | 21.2 | 24.3 | 28.3 | 24.9 | 0.11 | 86.2% |
| 3 | 20.3 | 24.0 | 30.3 | 27.1 | 0.07 | 85.8% |

As shown in the table, for polymeric nanomicelles prepared under the combined action of shear force and ultrasound, the results of three parallel tests were reproducible and stable.
Comparing Examples 26 and 25, stable polymeric nanomicelles can be produced under the simultaneous action of ultrasound with conditions that could not form polymeric nanomicelles.

**Example 27: Preparation of PTX/PEG-PLA polymeric micelle (first phase 50 ml/min, second phase 300 ml/min, under ultrasonication, 50 mL produced)**

First phase solution: PTX, PEG-PLA were co-dissolved in acetonitrile, PEG-PLA (50 mg/mL), PTX (15 mg/mL), filtration with 0.22 $\mu$m nylon filter membrane.
Second phase solution: water.
Spray hole diameter at the end of first pipeline Di(S)=0.25 mm;
Inner diameter of the second pipeline $D_2$(IN) =0.8 mm;
Inner diameter of the combined pipeline $D_3$(IN)=0.8 mm;

Outer diameter at the end of first pipeline $D_1(O)$=0.35 mm;
Combined phase length=360 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 50 | 300 |

Calculated based on the fluid in the circular pipeline, the combined phase Re=9176.
The first phase outlet flow velocity is: 17.0 m/s
The second phase flow velocity is: 11.6 m/s
FVR=1.46
Production quantity: 50 ml

| No. | D10 (nm) | D50 (nm) | D90 (nm) | Average diameter (nm) | PDI | Encapsulation efficiency |
|---|---|---|---|---|---|---|
| 1 | 26.4 | 48.1 | 92.7 | 45.1 | 0.20 | 86.0% |

Comparing Examples 26 and 27, it can be found that under the combined action of shear force and ultrasonic, polymeric nanomicelles with smaller particle sizes can also be prepared with different organic solvents and different organic phase flow velocitys, and the polymeric nanomicelles prepared have good stability (Figure 10).

**Example 28: Preparation of PTX/PEG-Phe-PLA polymeric micelle (first phase 14 ml/min, second phase 210 ml/min, without ultrasonication, 50 mL produced)**

First phase solution: PTX, PEG-Phe-PLA were co-dissolved in acetonitrile, PEG-Phe-PLA (50 mg/mL), PTX (15 mg/mL), filtration with 0.22 $\mu$m nylon filter membrane.
Second phase solution: water.
Spray hole diameter at the end of first pipeline Di(S)=0.25 mm;
Inner diameter of the second pipeline $D_2(IN)$ =0.8 mm;
Inner diameter of the combined pipeline $D_3(IN)$=0.8 mm;
Outer diameter at the end of first pipeline $D_1(O)$=0.35 mm;
Combined phase length=360 mm;
First phase solution temperature ti=25°C;
Second phase solution temperature $t_2$=25°C;

| First phase ml/min | Second phase ml/min |
|---|---|
| 14 | 210 |

Calculated based on the fluid in the circular pipeline, the combined phase Re=5872.
The first phase outlet flow velocity is: 4.75 m/s
The second phase flow velocity is: 7.43 m/s
FVR=0.64
Production quantity: 50 ml
The solution obtained without ultrasonication had more white flocculent precipitate, and the particle size could not be detected.

**Example 29: Preparation of PTX/PEG-Phe-PLA polymeric micelle (first phase 14 ml/min, second phase 210 ml/min, under ultrasonication, 50 mL produced)**

First phase solution: PTX, PEG-Phe-PLA were co-dissolved in acetonitrile, PEG-Phe-PLA (50 mg/mL), PTX (15 mg/mL), filtration with 0.22 $\mu$m nylon filter membrane.
Second phase solution: water.
Spray hole diameter at the end of first pipeline Di(S)=0.25 mm;

Inner diameter of the second pipeline $D_2(IN)$ =0.8 mm;
Inner diameter of the combined pipeline $D_3(IN)$=0.8 mm;
Outer diameter at the end of first pipeline $D_1(O)$=0.35 mm;
Combined phase length=360 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 14 | 210 |

Calculated based on the fluid in the circular pipeline, the combined phase Re=5872.
The first phase outlet flow velocity is: 4.75 m/s
The second phase flow velocity is: 7.43 m/s
FVR=0.64
Production quantity: 50 ml

| | D10 (nm) | D50 (nm) | D90 (nm) | Average diameter (nm) | PDI | Encapsulation efficiency |
|---|---|---|---|---|---|---|
| 1 | 18.7 | 21.3 | 24.3 | 22.3 | 0.07 | 88.2% |
| 2 | 18.7 | 21.4 | 24.4 | 24.2 | 0.05 | 88.8% |
| 3 | 19.0 | 21.8 | 24.8 | 23.7 | 0.10 | 88.5% |

As shown in the table, for polymeric nanomicelles prepared under the combined action of shear force and ultrasound, the results of three parallel tests were reproducible and stable.

Comparing Examples 29 and 28, stable polymeric nanomicelles can be produced under the simultaneous action of ultrasound with conditions that could not form polymeric nanomicelles.

**Example 30: Preparation of PEG-PLA polymer nanoparticle (first phase 50 ml/min, second phase 300 ml/min, without ultrasonication, 50 mL produced)**

First phase solution: PEG-PLA was dissolved in ethanol, PEG-PLA (50 mg/mL), filtration with 0.22 $\mu$m nylon filter membrane.

Second phase solution: water.
Spray hole diameter at the end of first pipeline Di(S)=0.25 mm;
Inner diameter of the second pipeline $D_2(IN)$ =0.8 mm;
Inner diameter of the combined pipeline $D_3(IN)$=0.8 mm;
Outer diameter at the end of first pipeline $D_1(O)$=0.35 mm;
Combined phase length=360 mm;
First phase solution temperature $t_1$=60°C;
Second phase solution temperature $t_2$=60°C;

| First phase ml/min | Second phase ml/min |
|---|---|
| 50 | 300 |

Calculated based on the fluid in the circular pipeline, the combined phase Re=9176.
The first phase outlet flow velocity is: 17.0 m/s
The second phase flow velocity is: 11.6 m/s
FVR=1.46
Production quantity: 50 ml

| No. | D10 (nm) | D50 (nm) | D90 (nm) | Average diameter (nm) | PDI | Encapsulation efficiency |
|---|---|---|---|---|---|---|
| 1 | 19.09 | 125.6 | 263.5 | 69.5 | 0.520 | - |

**Example 31: Preparation of PEG-PLA polymer nanoparticle (first phase 50 ml/min, second phase 300 ml/min, under ultrasonication, 50 mL produced)**

First phase solution: PEG-PLA was dissolved in ethanol, PEG-PLA (50 mg/mL), filtration with 0.22 $\mu$m nylon filter membrane.

Second phase solution: water.

Spray hole diameter at the end of first pipeline Di(S)=0.25 mm;

Inner diameter of the second pipeline $D_2$(IN) =0.8 mm;

Inner diameter of the combined pipeline $D_3$(IN)=0.8 mm;

Outer diameter at the end of first pipeline $D_1$(O)=0.35 mm;

Combined phase length=360 mm;

First phase solution temperature $t_1$=60°C;

Second phase solution temperature $t_2$=60°C;

Two phases combine in a 200W ultrasonic bath

| First phase ml/min | Second phase ml/min |
|---|---|
| 50 | 300 |

Calculated based on the fluid in the circular pipeline, the combined phase Re=9176.

The first phase outlet flow velocity is: 17.0 m/s

The second phase flow velocity is: 11.6 m/s

FVR=1.46

Production quantity: 50 ml

| No. | D10 (nm) | D50 (nm) | D90 (nm) | Average diameter (nm) | PDI | Encapsulation efficiency |
|---|---|---|---|---|---|---|
| 1 | 16.9 | 25.5 | 39.2 | 25.0 | 0.152 | - |

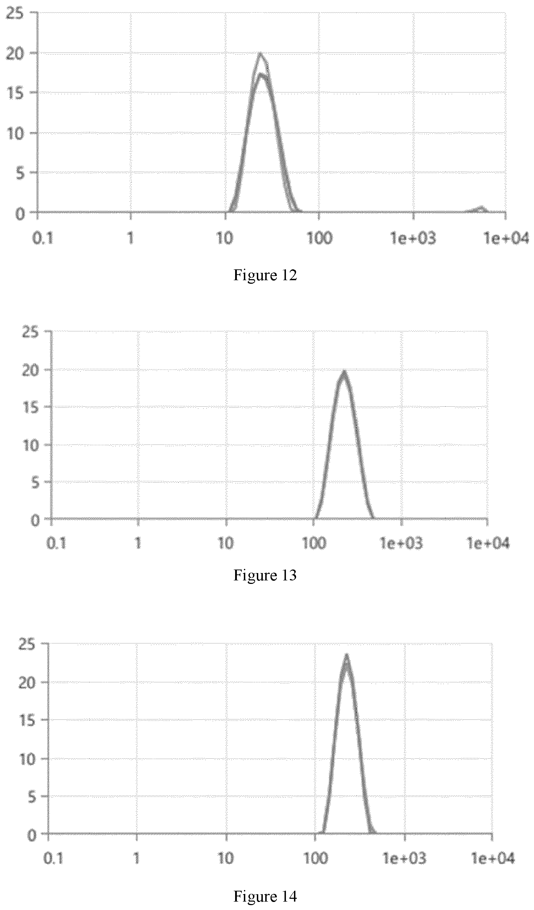

It can be seen from the comparison of Example 30 (Figure 11) and Example 31 (Figure. 12) that the particle size of the nanoparticles obtained without ultrasonic action is larger, and it shows a distribution with multiple peaks, larger polydispersity index and nonuniform in size. However, under the combined action of shear force and ultrasound, the prepared nanoparticles have a smaller particle size, a distribution with single peak, narrow polydispersity index and uniform particle size. These results indicate that the synergistic effect of ultrasound plays an important role in the preparation of nanoparticles with small particle size and narrow distribution.

**Example 32: Preparation of PLGA polymer nanoparticle (first phase 50 ml/min, second phase 300 ml/min, under ultrasonication, 50 mL produced)**

First phase solution: PLGA was dissolved in ethanol, filtration with 0.22 $\mu$m nylon filter membrane.

Second phase solution: water.

Spray hole diameter at the end of first pipeline Di(S)=0.25 mm;

Inner diameter of the second pipeline $D_2$(IN) =0.8 mm;

Inner diameter of the combined pipeline $D_3$(IN)=0.8 mm;

Outer diameter at the end of first pipeline $D_1$(O)=0.35 mm;

Combined phase length=360 mm;

First phase solution temperature $t_1$=60°C;

Second phase solution temperature $t_2$=60°C;

Two phases combine in a 200W ultrasonic bath

| First phase ml/min | Second phase ml/min |
|---|---|
| 50 | 300 |

Calculated based on the fluid in the circular pipeline, the combined phase Re=9176.

The first phase outlet flow velocity is: 17.0 m/s

The second phase flow velocity is: 11.6 m/s
FVR=1.46
Production quantity: 50 ml

| No. | D10 (nm) | D50 (nm) | D90 (nm) | Average diameter (nm) | PDI | Encapsulation efficiency |
|-----|----------|----------|----------|----------------------|-----|--------------------------|
| 1 | 154.1 | 226.9 | 337.8 | 218.5 | 0.063 | - |

As shown in the table, PLGA polymer nanoparticles can be prepared under the combined action of shear force and ultrasound, and the particle size distribution is narrow with a single peak (Figure 13).

**Example 33: Preparation of PTX/PLGA polymer nanoparticle (first phase 50 ml/min, second phase 300 ml/min, under ultrasonication, 50 mL produced)**

First phase solution: PTX and PLGA were dissolved in ethanol, filtration with 0.22 $\mu$m nylon filter membrane.
Second phase solution: water.
Spray hole diameter at the end of first pipeline Di(S)=0.25 mm;
Inner diameter of the second pipeline $D_2$(IN) =0.8 mm;
Inner diameter of the combined pipeline $D_3$(IN)=0.8 mm;
Outer diameter at the end of first pipeline $D_1$(O)=0.35 mm;
Combined phase length=360 mm;
First phase solution temperature $t_1$=25°C;
Second phase solution temperature $t_2$=25°C;
Two phases combine in a 200W ultrasonic bath

| First phase ml/min | Second phase ml/min |
|--------------------|---------------------|
| 50 | 300 |

Calculated based on the fluid in the circular pipeline, the combined phase Re=9176.
The first phase outlet flow velocity is: 17.0 m/s
The second phase flow velocity is: 11.6 m/s
FVR=1.46
Production quantity: 50 ml

| No. | D10 (nm) | D50 (nm) | D90 (nm) | Average diameter (nm) | PDI | Encapsulation efficiency |
|-----|----------|----------|----------|----------------------|-----|--------------------------|
| 1 | 165.6 | 230.9 | 326.0 | 225.9 | 0.027 | 80.2% |

As shown in the table, PTX/PLGA polymer nanoparticles can be prepared under the combined action of shear force and ultrasound, and the particle size distribution is narrow with a single peak (Figure 14).

**Example 34: Preparation of Polyl lipid nanoparticle (first phase 100 ml/min, second phase 200 ml/min, without ultrasonication, 50 mL produced)**

First phase solution: 32.31 mg ALC-0315 (CAS#:2036272-55-4), 4.08 mg DSPE-PEG2000 (CAS#: 147867-65-0), 7.08 mg HSPC (CAS#: 92128-87-5), 14.01 mg cholesterol were dissolved in ethanol, filtration with 0.22 $\mu$m nylon filter membrane.
Second phase solution: 5.71 mg Polyl (CAS#: 30918-54-8) in 3 mM citrate buffer solution (pH 4.0).
Spray hole diameter at the end of first pipeline Di(S)=0.25 mm;
Inner diameter of the second pipeline $D_2$(IN) =0.8 mm;
Inner diameter of the combined pipeline $D_3$(IN)=0.8 mm;
Outer diameter at the end of first pipeline $D_1$(O)=0.35 mm;
Combined phase length=360 mm;
First phase solution temperature $t_1$=25°C;
Second phase solution temperature $t_2$=25°C;

| First phase ml/min | Second phase ml/min |
|---|---|
| 100 | 200 |

Calculated based on the fluid in the circular pipeline, the combined phase Re=7865.
The first phase outlet flow velocity is: 34.0 m/s
The second phase flow velocity is: 9.94 m/s
FVR=3.4
Production quantity: 50 ml

| No. | D10 (nm) | D50 (nm) | D90 (nm) | Average diameter (nm) | PDI | Encapsulation efficiency |
|---|---|---|---|---|---|---|
| 1 | 42.6 | 84.7 | 624.8 | 89.6 | 0.41 | 35.3% |

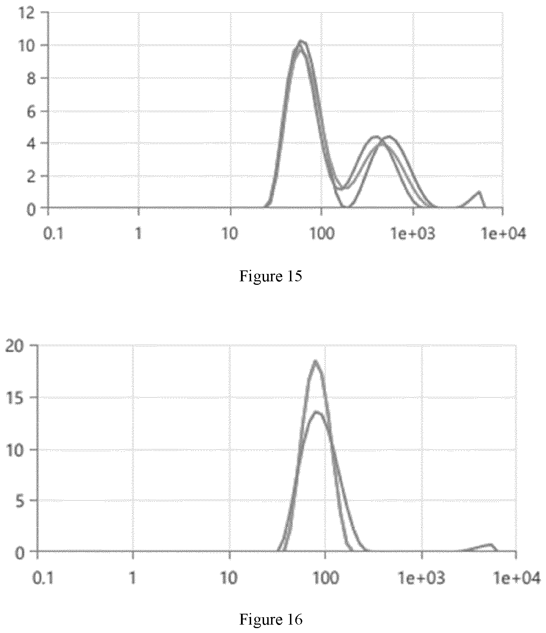

PolyI lipid nanoparticles prepared without ultrasonication shows a distribution with multiple peaks (Figure 15) and has low encapsulation efficiency.

**Example 35: Preparation of PolyI lipid nanoparticle (first phase 100 ml/min, second phase 200 ml/min, under ultrasonication, 50 mL produced)**

First phase solution: 32.31 mg ALC-0315, 4.08 mg DSPE-PEG2000, 7.08 mg HSPC, 14.01 mg cholesterol were dissolved in ethanol, filtration with 0.22 $\mu$m nylon filter membrane.
Second phase solution: 5.71 mg PolyI in 3 mM citric acid buffer (pH 4.0).
Spray hole diameter at the end of first pipeline Di(S)=0.25 mm;
Inner diameter of the second pipeline $D_2$(IN) =0.8 mm;
Inner diameter of the combined pipeline $D_3$(IN)=0.8 mm;
Outer diameter at the end of first pipeline $D_1$(O)=0.35 mm;
Combined phase length=360 mm;
First phase solution temperature $t_1$=25°C;
Second phase solution temperature $t_2$=25°C;
Two phases combine in a 200W ultrasonic bath

| First phase ml/min | Second phase ml/min |
|---|---|
| 100 | 200 |

Calculated based on the fluid in the circular pipeline, the combined phase Re=7865.
The first phase outlet flow velocity is: 34.0 m/s
The second phase flow velocity is: 9.94 m/s
FVR=3.4
Production quantity: 50 ml

| No. | D10 (nm) | D50 (nm) | D90 (nm) | Average diameter (nm) | PDI | Encapsulation efficiency |
|---|---|---|---|---|---|---|
| 1 | 53.9 | 83.5 | 133.3 | 85.7 | 0.21 | 78.9% |

As shown in the table, the PolyI lipid nanoparticle prepared under the combined action of shear force and ultrasonication showed a distribution with a single peak (Figure 16), narrow particle size distribution and high encapsulation efficiency.

**Comparative example 1: Preparation of SN-38/ICG nanoparticle (mixing followed by ultrasound)**

First phase solution: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
Second phase solution: water
Spray hole diameter at the end of first pipeline Di(S)=0.6mm;

Inner diameter of the second pipeline $D_2$(IN) =5.4mm;
Inner diameter of the combined pipeline $D_3$(IN)=5.4mm;
Outer diameter at the end of first pipeline $D_1$(O)=2 mm;
Outer diameter of the second pipeline $D_2$(O)=6 mm;
Outer diameter of the combined pipeline $D_3$(O)=6 mm;
Combined phase length=90 mm;
First phase solution temperature ti=25°C;
Second phase solution temperature $t_2$=25°C;
Two phases combine in a 200W ultrasonic bath

| First phase ml/min | Second phase ml/min |
|---|---|
| 11 | 193 |

Calculated based on the fluid in the circular pipeline, the combined phase Re=747, with SK type static mixer, static mixer size: 5.3mm*85 mm, with a total of 16 repeating spiral plates.
The first phase outlet flow velocity is: 0.648 m/s
The combined phase flow velocity is: 0.148 m/s
FVR=4.4
Production quantity: 20 ml
The solutions were mixed first according to the above conditions, and the resulting mixture was sonicated for 10 minutes (ultrasonic power: 200W, ultrasonic temperature 25°C)

| Ultrasound | D10 (nm) | D50 (nm) | D90 (nm) | Average diameter (nm) | PDI | Filtration encapsulation efficiency (SN-38) |
|---|---|---|---|---|---|---|
| mixing followed by ultrasonication | 36.7 ±5.6 | 162.8 ±10.7 | 272.8 ±55.0 | 1416±168 | 0.443± 0.089 | 53.2% |

Compared with (2) in Example 4, the particle size of the nanoparticles is slightly reduced, but the encapsulation efficiency cannot be improved.

**Comparative example 2: Preparation of SN-38/ICG nanoparticle (ultrasonication only)**

Solution A: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
45 ml of water was added to the flask, 5 ml of solution A was added under ultrasonication (ultrasonic power 200W), and was sonicated at 25 °C for 10 minutes. The resulting mixture has a lot of flocculent precipitate, the particle size can not be detected, and nanoparticles cannot be formed.

**Comparative example 3: Preparation of SN-38/ICG nanoparticle (ultrasonication first, then mix through a mixer)**

Solution A: ICG and SN-38 were dissolved in DMSO, SN-38 content: 3.394 wt.%, ICG content: 3.397 wt.%, SN-38 to ICG molar ratio was 2:1, filtration with 0.22 $\mu$m nylon filter membrane.
45 ml of water was added to the flask, 5 ml of solution A was added under ultrasonication (ultrasonic power 200W), and was sonicated at 25 °C for 10 minutes. The resulting mixture has a lot of flocculent precipitate. The mixture was mixed through mixer following conditions of (2) in Example 4, resulting in a cloudy mixture, and nanoparticles cannot be formed. The particle size can not be detected. The pipelines were blocked.
Although the specific embodiments of the present invention have been described above, those skilled in the art should understand that these are only illustrations, and that various changes or modifications may be made to these embodiments without deviating from the principle and substance of the present invention. Therefore, the scope of protection of the present invention is limited by the attached claims.

**Claims**

1.  A production system, which includes (a) a first pipeline, (b) a second pipeline, (f) an ultrasonic device, (c) a combined pipeline and (e) a (fluid) outlet;
    wherein, the first pipeline and the second pipeline are connected to the combined pipeline, the first pipeline is coaxial with the combined pipeline and the second pipeline is perpendicular to the combined pipeline; the first pipeline outlet is positioned within the combined pipeline; the first phase solution enters the combined pipeline through the first pipeline outlet, the second phase solution enters the combined pipeline through the second pipeline outlet; the ultrasonic device acts on part or the whole of combined pipeline; the first phase solution and the second phase solution are mixed in the combined pipeline to form a combined phase; the combined phase flows out through the outlet of the combined pipeline.

2.  The system of claim 1, wherein:

    (1) the core part of the production system includes: (a) a first pipeline; (b) a second pipeline; (c) a combined pipeline; (d) turbulent mixing device; (e) fluid outlet; (f) ultrasound devices with adjustable power; wherein, the first pipeline and the second pipeline are connected to the combined pipeline, the first phase solution enters the combined pipeline through the first pipeline outlet, the second phase solution enters the combined pipeline through the second pipeline outlet, the first phase solution and the second phase solution are mixed in the combined pipeline to form a combined phase; the power adjustable ultrasonic device acts on part or the whole of combined pipeline; the combined phase is fully mixed by a turbulent mixing device; after the mixing, the nanoparticles are collected into a suitable container through the outlet of the combined pipeline;
    (2) the mixing process of the first phase solution and the second phase solution is carried out under ultrasonication;
    (3) the nano-formulations are polymeric nanomicelles, nanoliposomes, lipid nanoparticles or small molecule nanoparticle assemblies.

3.  The system of any one of claim 1-2, wherein the production system fulfills one or more of the following:

    (1) the nano-formulations are selected from one or more of polymeric nanomicelles, polymer nanoparticles, nanoliposomes, lipid nanoparticles and small molecule nanoparticle assemblies;
    (2) the first pipeline outlet is a spray hole with a certain shape and diameter, and the first phase solution passes through the first pipeline and enters the combined pipeline through the spray hole.

4.  The system of any one of claim 1-2, wherein the production system fulfills one or more of the following:

    (1) the combined pipeline length is selected from 6 to 120 cm, such as 9 cm or 36 cm;
    (2) the ratio of the combined pipeline length to the combined pipeline inner diameter is (16-450):1, such as 16.7: 1, 30: 1 or 450:1;
    (3) the first pipeline outer diameter D1(O) is 0.35 to 2 mm, such as 0.35 mm, 1 mm or 2 mm;
    (4) the spray hole diameter D1(S) at the end of first pipeline is selected from 0.03-5 .0 mm; preferably 0.2 to 0.6 mm, such as 0.2 mm, 0.25 mm, 0.3 mm, 0.4 mm or 0.6 mm;
    (5) the second pipeline outer diameter D2(O) is 6 mm;
    (6) the second pipeline inner diameter D2(IN) is selected from 0.3-50.0 mm; preferably 0.8 to 5.4 mm, such as 0.8 mm, 3.0 mm or 5.4 mm;
    (7) the combined pipeline outer diameter D3(O) is 6 mm;
    (8) the combined pipeline inner diameter D3(IN) is selected from 0.03-5.0 mm; preferably 0.8 to 5.4 mm, such as 0.8 mm, 3.0 mm or 5.4 mm;
    (9) the second pipeline inner diameter D2(IN) is the same as the combined pipeline inner diameter D3(IN);
    (10) the ratio of the spray hole diameter D1(S) at the end of first pipeline to the combined pipeline inner diameter D3(IN) can be 1:(2-50), such as 1:3.2, 1:7.5, 1:9 or 1: 18;
    (11) the mixing is turbulent mixing; the turbulent mixing can be achieved by adding a turbulent mixing device in the combined pipeline; there can be one or more turbulent mixing devices;
    (12) the ultrasonic devices are ultrasound devices with adjustable power.

5.  The system of claim 4, wherein the production system fulfills one or more of the following:

    (1) the materials used in the first pipeline, the second pipeline, the combined pipeline, the turbulent mixing device, and the fluid outlet are each selected from one or more of stainless steel, polytetrafluoroethylene, polyethylene, polypropylene, latex, silicone, or other polymer materials;

(2) the turbulent mixing achieved by turbulent mixing device includes: (a) increasing the fluid flow velocity; (b) changing the degree of pipeline twisting; (c) adding baffles or specialized objects within the pipeline, such as a static mixer;

the Reynolds number calculated based on the fluid in the circular tube is selected from 500-100000;

(3) the turbulent mixing device is a device that mixes the first phase solution and the second phase solution to reach a turbulent flow state, such as a static mixer; the static mixer can be selected from one or more of SV type static mixer, SX type static mixer, SL type static mixer, SH type static mixer and SK type static mixer, preferably SK type static mixer;

(4) the quantity of flow Q1 of the first phase solution through the first pipeline is selected from 1-1000 ml/min; the temperature t1 of the first phase solution is selected from 0-90°C; the quantity of flow Q2 of the second phase solution through the second pipeline is selected from 10-10000 ml/min; the temperature t2 of the second phase solution is selected from 0-90°C;

(5) the ultrasonic frequency of the power adjustable ultrasonic device is 15 kHz-1.0 MHz, preferably 15 kHz-40 kHz, more preferably 19 kHz-40 kHz; the ultrasonic power range is 0.1-20 kW, preferably 100-1000 W.

6. The system of any one of claim 1-2, wherein the production system fulfills one or more of the following:

(1) the polymeric nanomicelle component is selected from an amphiphilic polymer and an antitumor drug; the amphiphilic polymer is selected from PEG-PLA, PEG-PCL, PEG-linker-PLA or PEG-linker-PCL, wherein, the linker is a linker selected from C1-C30 small molecule fragment; and the number average molecular weight of PEG is 400-20000 polyethylene glycol segments or mono-protected polyethylene glycol segments;

(2) the nanoliposomes are blank liposomes without drug encapsulation or liposomes with drug encapsulation;

(3) the lipid nanoparticles are lipid nanoparticles encapsulating antitumor drugs;

(4) the small molecule nanoassemblies are selected from antitumor drug/photosensitizer nanoassemblies, antitumor drug/antitumor drug nanoassemblies, antitumor drug/other drug (e.g., curcumin) nanoassemblies, antitumor drug/excipient (e.g., amphiphilic polymer PEG-PLA, DSPE-PEG or PLGA polymer) nanoassemblies, two or more drug nanoassemblies (e.g., SN38 and irinotecan), or small molecule drug/excipient nanoassemblies.

7. The system of claim 6, wherein the production system fulfills one or more of the following:

(1) the antitumor drug is selected from one or more of abemaciclib, abiraterone, abrocitinib, acalabrutinib, afatinib, aldesleukin, alectinib, alflutinib, almonertinib, altretamine, amcenestrant, aminoglutethimide, amsacrine, anastrozole, anlotinib, apalutamide, apatinib, arzoxifene, asciminib, asparaginase, avapritinib, avitinib, axitinib, azacitidine, baricitinib, belinostat, bendamustine, bexarotene, bicalutamide, bicyclol, binimetinib, bleomycin, boanmycin, bortezomib, bosutinib, brigatinib, buserelin, busulfan, cabazitaxel, cabozantinib, calaspargase, calicheamycin, capecitabine, capmatinib, carboplatin, carfilzomib, carmustine, carmofur, cedazuidine, ceritinib, cetrorelix, chidamide, chlorambucil, cisplatin, cladribine, clofarabine, cobimetinib, colchicine, copanlisib, crizotinib, cyclophosphamide, cytarabine, dabrafenib, dacarbazine, dacomitinib, dactinomycin, dalpiciclib, darolutamide, dasatinib, daunorubicin, decitabine, degarelix, delgociclib, denileukin, deruxtecan, docetaxel, donafenib, doxorubicin, duvelisib, enasidenib, encorafenib, ensartinib, entrectinib, enzalutamide, enzastaurin, elacestrant, epirubicin, erdafitinib, eribulin, erlotinib, estradiol, estramustine, etoposide, everolimus, exemestane, fasudil, fedatinib, filgotinib, floxuridine, fludarabine, flumatinib, fluorouracil, flutamide, fluzoparib, formestane, fostamatinib, fruquintinib, fulvestrant, gefitinib, gemcitabine, gilteritinib, giredestrant, glasdegib, goserelin, histrelin, hydroxyurea, ibrutinib, ibudilast, icaritin, icotinib, idarubicin, idelalisib, ifosfamide, imatinib, imiquimod, infigratinib, ingenol mebutate, interferon alfa-2b, irinotecan, ivosidenib, ixabepilone, ixazomib, lanreotide, lapatinib, larotrectinib, lenalidomide, lenvatinib, letrozole, leucovorin, leuprolide, lomustine, lonafamib, lorlatinib, lurbinctedin, maytansine, mechlorethamine, medroxyprogesterone, megestrol, melphalan, melphlan flufenamide, mercaptopurine, methotrexate, methoxsalen, methylprednisolone, midostaurin, mitomycin, mitotane, mitoxantrone, mitozolomide, mobocertinib, monomethylauristatin E, monomethylauristatin F, nelarabine, nandrolone, neratinib, nearsudil, nilotinib, nilutamide, nintedanib, niraparib, octreotide, olaparib, olmutinib, omacetaxine, orelabrutinib, osimertinib, oxaliplatin, paclitaxel, pacritinib, palbociclib, pamidronate, pamiparib, panobinostat, pazopanib, peficitinib, pegaptanib, pegaspargase, peginteferon alfa-2b, pemigatinib, pemetrexed, pentetreotide, pentostatin, pexidartinib, phenoxybenzamine, pidotimod, plinabulin, plitidepsin, pomalidomide, ponatinib, porfimer, pralatrexate, pralsetinib, prednisolone, procarbazine, pyrotinib, quizartinib, radotinib, raloxifene, raltitrexed, regorafenib, ribociclib, rintatolimod, ripretinib, romidepsin, rucaparib, ruxolitinib, savolitinib, selinexor, selpercatinib, selumetinib, sonidegib, sorafenib, sotorasib, streptozocin, sunitinib, surufatinib, talazoparib, tamoxifen, tazemetostat, tegafur, temozolomide, temsirolimus, teniposide, tepotinib, teprenone, thalidomide, thioguanine, thiotepa, thyrotropin alfa, tipiracil, tipifamib, tirabrutinib, tirbanibulin, tivozanib, trametinib, tofacitinib, topotecan, toremifene, trabectedin, tretinoin, trifluride, trilaciclib, triptorelin, tucatinib, upadacitinib, umbralisib, utidelone, uroacitide, valrubicin, vandetanib, vemurafenib, venetoclax, vinblastine, vincristine, vindesine, vinflunine, vinorelbine, vismodegib, vorinostat, zanubrutinib, zoledronic acid, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, 7-N,N-dimethylglycylpaclitaxel, 7-L-alanylacetaxel, larotaxel, camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, pirarubicin, aclacinomycin, sirolimus, tacrolimus, progesterone, estrogen, rapamycin, mithramycin, harringtonine or curcumin;

the antitumor drug is preferably selected from one or more of camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, topotecan, deruxtecan, paclitaxel, docetaxel, cabazitaxel, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, 7-N,N-dimethylglycylpaclitaxel, 7-L-alanylacetaxel, larotaxel, doxorubicin, epirubicin, daunorubicin, pirarubicin, aclacinomycin, etoposide, teniposide, vinblastine, vincristine, vinorelbine, vindesine, maytansine, curcumin, harringtonine, homoharringtonine, gemcitabine, capecitabine, fludarabine, cladribine, pemetrexed, bortezomib, carfilzomib, ixazomib, carmustine, fluorouracil, cytarabine, cyclosporine A, eribulin, trabectedin, gefitinib, erlotinib, lapatinib, afatinib, dacomitinib, vandetanib, neratinib, osimertinib, imatinib, sorafenib, sunitinib, lapatinib, dasatinib, olaparib, niraparib, rucaparib, fluzoparib, pamiparib, veliparib, talazoparib, apatinib, palbociclib, abemaciclib, ribociclib;

(2) the photosensitizers include cyanine molecules, porphyrin molecules, porphyrin precursors, phthalocyanine molecules and chlorin molecules;

wherein, the cyanine molecules are preferably selected from one or more of indocyanine green (IR780), new indocyanine green (IR820), indocyanine green and indocyanine green analogs; the porphyrin molecule is preferably selected from hematoporphyrin monomethyl ether; the porphyrin precursor is preferably selected from one of 5-aminolevulinic acid and 5-aminolevulinic acid esters; the phthalocyanine molecule is preferably selected from one or more of copper phthalocyanine, cobalt phthalocyanine, aluminum phthalocyanine, nickel phthalocyanine, calcium phthalocyanine, sodium phthalocyanine, magnesium phthalocyanine, zinc phthalocyanine, indium phthalocyanine, oxytitanium phthalocyanine, manganese phthalocyanine or phthalocyanine derivatives; the chlorin molecules are preferably selected from one or more of chlorins, talaporfin, verteporfin, temoporfin, rostaporfin, porfimer sodium, hemoporfin and HPPH.

8. The system of claim 3, wherein the production system fulfills one or more of the following:

(1) the polymeric nanomicelles are PTX/PEG-PLA polymeric micelles or PTX/PEG-Phe-PLA polymeric micelles;
(2) the nanoliposomes are HSPC/CHOL/DSPE-PEG blank liposomes or PTX/HSPC/CHOL/DSPE-PEG liposomes; wherein, the molar ratio of HSPC/CHOL/DSPE-PEG is 56:38:5 or 89:57:4;
(3) the lipid nanoparticle is a PolyI lipid nanoparticle, such as Poly I/ALC-0315/DSPE-PEG2000/HSPC/cholesterol;
(4) the polymer nanoparticles are PEG-PLA polymer nanoparticles, PLGA polymer nanoparticles or PTX/PLGA polymer nanoparticles;
(5) the small molecule nanoassembly is an anti-tumor drug/photosensitizer nanoassembly, preferably SN-38/ICG nanoparticles, PTX/ICG nanoparticles, curcumin/CPT11 nanoparticles or SN-38/CPT11 nanoparticles.

9. A production method for producing nano-formulations, which includes the following steps: in the system of any one of claim 1-8, the first phase solution and the second phase solution are mixed under ultrasonication, nano-formulations are collected from combined phase through the fluid outlet;

A. when the nano-formulations are polymeric nanomicelles, the solvent in the first phase solution is a good solvent for an anti-tumor drug or its pharmaceutically acceptable salt, and the solute is (1) the anti-tumor drug or its pharmaceutically acceptable salt, or (2) the anti-tumor drug or its pharmacologically acceptable salt and polymer;

the solvent in the second phase solution is an anti-solvent of the anti-tumor drug or its pharmaceutically acceptable salt, and the solute is (1) absent, or (2) polymer;

then the solute in the first phase solution is an anti-tumor drug or its pharmaceutically acceptable salt and polymer, the solute in the second phase solution does not exist;

when the solute in the first phase solution is an anti-tumor drug or its pharmaceutically acceptable salt, the solute in the second phase solution is polymer;

B. when the nano-formulations are polymer nanoparticles, the solvent of the first phase solution is a good solvent for the polymer, and the solute is (1) polymer, or (2) polymer and anti-tumor drug;

the second phase solution is water or water containing 0.5% PVA;

C. then the nano-formulations are nanoliposomes, the solvent of the first phase solution is a good solvent for the lipid component, and the solute is (1) the lipid component of the liposome, or (2) the lipid component of the liposome and the anti-tumor drug;

the second phase solution is water or aqueous buffer solution with a certain pH value and a certain osmotic pressure;

D. when the nano-formulations are lipid nanoparticles, the solvent of the first phase solution is a good solvent for the lipid component, and the solute is the lipid component of the lipid nanoparticle;

the solvent of the second phase solution is aqueous buffer solution with a certain pH value and a certain osmotic pressure; the solute is anti-tumor drug;

E. when the nano-formulations are anti-tumor drug/photosensitizer nanoassembly, the solvent in the first-phase solution is a good solvent of the anti-tumor drug or its pharmaceutically acceptable salt, and the solute is (1) the anti-tumor drug or its pharmaceutically acceptable salt and the photosensitizer, or (2) the anti-tumor drug or its pharmaceutically acceptable salt;

the solvent in the second phase solution is an anti-solvent of the anti-tumor drug or its pharmaceutically acceptable salt, and the solute is (1) absent, or (2) a photosensitizer;

when the solute in the first phase solution is an anti-tumor drug or its pharmaceutically acceptable salt and a photosensitizer, the solute in the second phase solution does not exist;

when the solute in the first phase solution is an anti-tumor drug or its pharmaceutically acceptable salt, the solute in the second phase solution is a photosensitizer.

10. The method of claim 9, wherein the production method includes any of the following:

a. preparation method for the continuous production of polymeric nanomicelles, and the method includes:

(1) production system according to any of claims 1-5;
(2) amphiphilic polymer according to claim 6;
(3) anti-tumor drug according to claim 7;
(4) one or more of the anti-tumor drugs or their pharmaceutically acceptable salts are dissolved in a first phase solvent, and the solvent for the first phase solution is a good solvent for the anti-tumor drugs or their pharmaceutically acceptable salts;
(5) one or more of polymers are dissolved in a second phase solvent, and the solvent for the second phase solution is an anti-solvent for anti-tumor drugs or their pharmaceutically acceptable salts;
(6) the first phase solution with the quantity of flow Q1 and the second phase solution with the quantity of flow Q2 are mixed in the combined phase; under the action of turbulent shear and ultrasound at the same time, the two-phase solutions mix rapidly to form a mixed solvent of the first phase and the second phase and produce a stably dispersed polymeric nanomicelles encapsulating anti-tumor drug with a certain particle size and distribution coefficient;

b. preparation method for continuous production of polymeric nanomicelles, and the method includes:

(1) production system according to any of claims 1-5;
(2) amphiphilic polymer according to claim 6;
(3) anti-tumor drug according to claim 7;
(4) one or more of the anti-tumor drugs or their pharmaceutically acceptable salts and one or more of the amphiphilic polymers are dissolved in a first phase solvent, and the solvent for the first phase solution is a good solvent for the anti-tumor drugs or their pharmaceutically acceptable salts;
(5) the solvent for the second phase solution is an anti-solvent for anti-tumor drugs or their pharmaceutically acceptable salts;

(6) the first phase solution with the quantity of flow Q1 and the second phase solution with the quantity of flow Q2 are mixed in the combined phase; under the action of turbulent shear and ultrasound at the same time, the two-phase solutions mix rapidly to form a mixed solvent of the first phase and the second phase and produce a stably dispersed polymeric nanomicelles with a certain particle size and distribution coefficient;

c. preparation method for continuous production of nanoliposomes, and the method includes:

(1) production system according to any of claims 1-6;
(3) the lipid component of the liposome is dissolved in the first phase, and the solvent used in the first phase solution is a good solvent for the lipid component;
(4) the second phase solution is selected from water, aqueous buffer solution with a certain pH value and a certain osmotic pressure;
(5) the first phase solution with the quantity of flow Q1 and the second phase solution with the quantity of flow Q2 are mixed in the combined phase; under the action of turbulent shear and ultrasound at the same time, the two-phase solutions mix rapidly to form a mixed solvent of the first phase and the second phase and produce a stably dispersed blank liposomes with a certain particle size and distribution coefficient;

d. preparation method for continuous production of anti-tumor drug/photosensitizer nanoassemblies, and the method includes:

(1) production system according to any of claims 1-6;
(2) anti-tumor drugs and photosensitizers according to claim 7;
(3) one or more of the anti-tumor drugs or their pharmaceutically acceptable salts are dissolved in a first phase solvent;
(4) one or more of photosensitizers are dissolved in a second phase solvent, and the solvent for the second phase solution is an anti-solvent for anti-tumor drugs or their pharmaceutically acceptable salts;
(5) the first phase solution with the quantity of flow Q1 and the second phase solution with the quantity of flow Q2 are mixed in the combined phase; under the action of turbulent shear and ultrasound at the same time, the two-phase solutions mix rapidly to form a mixed solvent of the first phase and the second phase and produce a stably dispersed anti-tumor drug/photosensitizer nanoassembly with a certain particle size and distribution coefficient;

e. preparation method for continuous production of anti-tumor drug/photosensitizer nanoassemblies, and the method includes:

(1) production system according to any of claims 1-6;
(2) anti-tumor drugs and photosensitizers according to claim 7;
(3) one or more of the anti-tumor drugs or their pharmaceutically acceptable salts and one or more of the photosensitizers are dissolved in a first phase solvent, and the solvent for the first phase solution is a good solvent for the anti-tumor drugs or their pharmaceutically acceptable salts;
(4) the solvent for the second phase solution is an anti-solvent for anti-tumor drugs or their pharmaceutically acceptable salts;
(5) the first phase solution with the quantity of flow Q1 and the second phase solution with the quantity of flow Q2 are mixed in the combined phase; under the action of turbulent shear and ultrasound at the same time, the two-phase solutions mix rapidly to form a mixed solvent of the first phase and the second phase and produce a stably dispersed anti-tumor drug/photosensitizer nanoassembly with a certain particle size and distribution coefficient.

**11.** The method of any one of claim 9-10, wherein the production method fulfills one or more of the following:

(1) the temperature of the first phase solution is 0-90°C, such as 25°C or 60°C;
(2) the temperature of the second phase solution is 0-90°C, such as 25°C or 60°C;
(3) the fluid Reynolds number Re of the combined phase is 700-9500 (e.g., 747, 2884, 3868, 5158, 5505, 5872, 6623, 7865 or 9176), preferably 3000-7000 (e.g., 3868, 5158 or 6623);
(4) the flow velocity ratio FVR between the first phase solution and the combined phase is 0.4-6, such as 0.49, 0.64, 0.93, 1.46, 3.38, 3.4, 4.4 or 5.2;
(5) the ultrasound is an ultrasonic water bath, and the ultrasonic power is 200W;

(6) the solvent used in the first phase solution and the second phase solution is water, aqueous buffer solution with a certain pH value, or an organic solvent miscible with water, and further, the organic solvent is one or more of methanol, ethanol, propanol, isopropanol, butanol, isobutanol, tert-butanol, DMF, DMAc, HMPA, N-methylpyrrolidone, DMSO, butyl sulfone, tetramethylene sulfone, THF, 2-methyltetrahydrofuran, acetonitrile, acetone, ethylene glycol, ethylene glycol methyl ether, ethylene glycol ethyl ether, dioxane, formic acid, acetic acid, hydroxypropionic acid, ethylamine, ethylenediamine, glycerol or pyridine;

(7) the quantity of flow Q1 of the first phase solution through the first pipeline is selected from 10-100 ml/min, such as 10, 11, 14, 50, 60, 80 or 100;

(8) the quantity of flow Q2 of the second phase solution through the second pipeline is selected from 100-1300 ml/min, such as 100, 193, 200, 210, 300, 936, 890 or 1248.

**12.** The method of any one of claim 9-10, wherein the production method fulfills one or more of the following:

(1) when the nano-formulation is polymeric nanomicelle, the concentration of the anti-tumor drug in the first phase solution ranges from 0.1-200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml, more preferably 10-20 mg/ml, such as 15 mg/ml;

(2) when the nano-formulation is polymeric nanomicelle, the concentration of the polymer in the first phase solution or the second phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml, such as 50 mg/ml;

(3) when the nano-formulation is nanoliposome, the concentration of the lipid component in the first phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml;

(4) when the nano-formulation is anti-tumor drug/photosensitizer nanoassembly, the molar ratio of the anti-tumor drug or its pharmaceutically acceptable salt to the photosensitizer is (1-15):1, such as 1:1, 2:1, 5:1, 6:1, 7:1, 8:1, 10:1 or 15:1, preferably 2:1;

(5) when the nano-formulation is anti-tumor drug/photosensitizer nanoassembly, the concentration of the anti-tumor drug in the first phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml, such as 40 mg/ml, 50 mg/ml or 100 mg/ml;

(6) when the nano-formulation is anti-tumor drug/photosensitizer nanoassembly, the concentration of the photosensitizer in the first phase solution or the second phase solution ranges from 0.1 to 200 mg/ml, for example, it can be 0.1 mg/ml, 1 mg/ml, 5 mg/ml, 10 mg/ml, 20 mg/ml, 40 mg/ml, 60 mg/ml, 80 mg/ml, 100 mg/ml, 120 mg/ml, 140 mg/ml, 160 mg/ml, 180 mg/ml, 200 mg/ml, preferably 10-100 mg/ml, such as 40 mg/ml, 50 mg/ml or 150 mg/ml.

**13.** The method of any one of claim 9-10, wherein the production method fulfills one or more of the following:

(1) when the nano-formulation is polymeric nanomicelle, the solvent used in the first phase solution is nitrile solvent or alcohol solvent, such as acetonitrile or ethanol;

(2) when the nano-formulation is polymeric nanomicelle, the solvent used in the second phase solution is water;

(3) when the nano-formulation is liposome, the solvent used in the first phase solution is alcohol solvent, such as ethanol;

(4) when the nano-formulation is liposome, the solvent used in the second phase solution is water or aqueous solution of ammonium sulfate, preferably water or an aqueous solution of 120 mM ammonium sulfate;

(5) when the nano-formulation is polymer nanoparticle, the solvent used in the first phase solution is alcohol solvent or chlorinated alkane solvent, such as ethanol or methylene chloride;

(6) when the nano-formulation is polymer nanoparticle, the solvent used for the second phase solution is water or water containing 0.5% PVA;

(7) when the nano-formulation is anti-tumor drug/photosensitizer nanoassembly, the solvent used in the first phase solution is sulfoxide solvent, alcohol solvent, such as dimethyl sulfoxide or methanol;

(8) when the nano-formulation is anti-tumor drug/photosensitizer nanoassembly, the solvent used in the second phase solution is water;

(9) when the nano-formulation is lipid nanoparticle, the first phase solution is alcohol solvent, such as ethanol;

(10) when the nano-formulation is lipid nanoparticle, the second phase solution is a citric acid buffer (pH 4.0).

14. The method of any one of claim 9-10, wherein the particle size of nano-formulations is less than 1000 nm; preferably less than 500 nm; more preferably less than 200 nm; such as 20-200 nm.

15. The method of any one of claim 9-10, wherein the polydispersity index of nano-formulations is less than 0.4.

16. A continuous production method for SN-3 8/indocyanine green nanoassemblies, and the method includes:

(1) production system according to any of claims 1-5;
(2) SN-38 and indocyanine green are dissolved in the first phase solution, wherein the solvent used in the first phase solution is a good solvent for SN-38 and indocyanine green;
(3) the second phase solution is an anti-solvent for anti-tumor drugs or their pharmaceutically acceptable salts;
(4) the first phase solution with the quantity of flow Q1 and the second phase solution with the quantity of flow Q2 are mixed in the combined phase; under the action of turbulent shear and ultrasound at the same time, the two-phase solutions mix rapidly to form a mixed solvent of the first phase and the second phase and produce a stably dispersed SN-38/indocyanine green nanoassembly with a certain particle size and distribution coefficient.

17. The method of claim 16, wherein the solvent used in the first phase solution and the second phase solution is water, aqueous buffer solution with a certain pH value or an organic solvent miscible with water, and further, the organic solvent is one or more of methanol, ethanol, propanol, isopropanol, butanol, isobutanol, tert-butanol, DMF, DMAc, HMPA, N-methylpyrrolidone, DMSO, butyl sulfone, tetramethylene sulfone, THF, 2-methyltetrahydrofuran, acetonitrile, acetone, ethylene glycol, ethylene glycol methyl ether, ethylene glycol ethyl ether, dioxane, formic acid, acetic acid, hydroxypropionic acid, ethylamine, ethylenediamine, glycerol or pyridine.

Inner diameter of the second pipeline $D_2(IN)$=5.4 mm;
Outer diameter of the second pipeline $D_2(O)$=6 mm;

Inner diameter of the combined
pipeline $D_3(IN)$=5.4 mm;

Ultrasonic bath

Outer diameter of the
combined pipeline $D_3(O)$=6 mm;

Combined pipeline

First pipeline

Outer diameter of a tail end of the first pipeline $D_1(O)$=2 mm;
Aperture of a spray hole in the tail end of the first pipeline $D_1(S)$=0.3 mm;

Figure 1

Inner diameter of the second pipeline $D_2(IN)$=5.4 mm;
Outer diameter of the second pipeline $D_2(O)$=6 mm;

Inner diameter of the combined
pipeline $D_3(IN)$=5.4 mm;

Ultrasonic bath

Outer diameter of the
combined pipeline $D_3(O)$=6 mm;

Combined pipeline

First pipeline

Outer diameter of a tail end of the first pipeline
$D_1(O)$=2 mm;
Aperture of a spray hole in the tail end of the
first pipeline $D_1(S)$=0.3 mm;

5.3 mm*85 mm
16 elements SK static mixer

Figure 2

Without ultrasonication

With ultrasonication

Figure 3

Example 5

Figure 4

Figure 5

Figure 6

Figure 7

Figure 8

Figure 9

Figure 10

Figure 11

Figure 12

Figure 13

Figure 14

Figure 15

Figure 16

# EP 4 450 045 A1

## INTERNATIONAL SEARCH REPORT

| | International application No. |
|---|---|
| | **PCT/CN2022/138806** |

**A. CLASSIFICATION OF SUBJECT MATTER**

A61J 3/00(2006.01)i;A61K 9/08(2006.01)i;A61K 9/107(2006.01)i;A61K 9/127(2006.01)i;A61K 31/4745(2006.01)i;A61K 41/00(2020.01)i;A61K 45/00(2006.01)i;A61P 35/00(2006.01)i;B82Y 5/00(2011.01)i

According to International Patent Classification (IPC) or to both national classification and IPC

**B. FIELDS SEARCHED**

Minimum documentation searched (classification system followed by classification symbols)

IPC:A61J A61K A61P B82Y

Documentation searched other than minimum documentation to the extent that such documents are included in the fields searched

Electronic data base consulted during the international search (name of data base and, where practicable, search terms used)

CNABS; CNKI; CNTXT; VCN; ENTXTC; ENTXT: 上海弼领, 张富尧, 万家勋, 纳米, 胶束, 颗粒, 管, 垂直, 同轴, 抗癌, 抗肿瘤, 聚合物, 超声, 湍流, 溶剂, 反溶剂, nano+, micelle, particle, tube, vertical, coaxial, anti-cancer, anti-tumor, polymer, ultrasound, turbulent, solvent, anti-solvent

**C. DOCUMENTS CONSIDERED TO BE RELEVANT**

| Category* | Citation of document, with indication, where appropriate, of the relevant passages | Relevant to claim No. |
|---|---|---|
| X | WO 2020263179 A1 (NATIONAL UNIVERSITY OF SINGAPORE) 30 December 2020 (2020-12-30) <br> description, p. 1, line 11 to p. 44, line 21, and figures 1A-8 | 1-17 |
| A | CN 111819217 A (SHANGHAI SELECTION BIOSCIENCE LLC.) 23 October 2020 (2020-10-23) <br> entire document | 1-17 |
| A | CN 112236459 A (GRAYBUG VISION, INC.) 15 January 2021 (2021-01-15) <br> entire document | 1-17 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 February 2023** | **09 March 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
Information on patent family members

International application No.

**PCT/CN2022/138806**

| Patent document cited in search report | | | Publication date (day/month/year) | Patent family member(s) | Publication date (day/month/year) |
|---|---|---|---|---|---|
| WO | 2020263179 | A1 | 30 December 2020 | None | |
| CN | 111819217 | A | 23 October 2020 | None | |
| CN | 112236459 | A | 15 January 2021 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111522472X
- CN 201680013882
- US 10940118 B2
- CN 108137819
- CN 108542894

**Non-patent literature cited in the description**

- *Langmuir,* 2012, vol. 28, 3633
- *Pharm Res.,* 2016, vol. 33, 404-416
- *Expert Opin. Drug Deliv.,* 2009, vol. 6, 865
- *Physical Review Letters,* 2003, vol. 91, 118301
- *AIChE Journal,* 2003, vol. 49, 2264
- *Mol. Pharm.,* 2013, vol. 10, 4367
- *Angew. Chem. Int. Ed. Engl,* 2021, vol. 60, 15590
- *CHEMICAL ABSTRACTS,* 2036272-55-4
- *CHEMICAL ABSTRACTS,* 147867-65-0
- *CHEMICAL ABSTRACTS,* 92128-87-5
- *CHEMICAL ABSTRACTS,* 30918-54-8